# FASCICULE DE BREVET EUROPEEN

(11) **EP 1 511 471 B1**
(45) Date de publication et mention de la délivrance du brevet: **02.02.2011**
(21) Numéro de dépôt: 03747151.3
(22) Date de dépôt: 23.04.2003
(51) Int. Cl.: A61K 9/51, B01J 13/00

(54) **SUSPENSION COLLOIDALE DE PARTICULES SUBMICRONIQUE DE VECTORISATION DE PRINCIPES ACTIFS ET LEUR MODE DE PREPARATION**
KOLLOIDALE SUSPENSION VON TEILCHEN IM SUBMIKRON-BEREICH ZUR ABGABE VON WIRKSTOFFEN UND VERFAHREN ZU IHRER HERSTELLUNG
COLLOIDAL SUSPENSION OF SUBMICRONIC PARTICLES FOR DELIVERING ACTIVE PRINCIPLES AND METHOD FOR PREPARING SAME

(30) Priorité: 26.04.2002 FR 0205312
(43) Date de publication de la demande: 09.03.2005
(73) Titulaire: FLAMEL TECHNOLOGIES, 69200 Vénissieux (FR)
(72) Inventeur: Caillol, Sylvain, 75017 Paris (FR); Meyrueix, Rémi, 69009 Lyon (FR); Bryson, Nathan, 69390 Millery (FR); Soum, Alain, 33607 Pessac Cedex (FR); Soula, Gérard, 69330 Meyzieu (FR); Mingotaud, Anne-Françoise, Lab. IMRCP, Bât 221, 31062 Toulouse Cedex (FR)
(74) Mandataire: Fleurance, Raphaël
(86) Numéro de dépôt international: PCT/FR2003/001278
(87) Numéro de publication internationale: WO 2003/090727

(56) Documents cités:
- WO-A-02/28521
- FR-A- 2 786 098
- GONSALVES K E ET AL: "SYNTHESIS AND SURFACE CHARACTERIZATION OF FUNCTIONALIZED POLYlACTIDE COPOLYMER MICROPARTICLES" BIOMATERIALS, ELSEVIER SCIENCE PUBLISHERS BV., BARKING, GB, vol. 19, no. 16, 1 août 1998 (1998-08-01), pages 1501-1505, XP000668516 ISSN: 0142-9612 cité dans la demande

## Description

Le domaine de la présente invention est celui des Particules de Vectorisation (**PV**), utiles pour l'administration de principes actifs (**PA**). Ces derniers sont, de préférence, des médicaments ou des nutriments pour l'administration à un organisme animal ou humain par voie orale ou nasale, vaginale, oculaire, sous-cutanée, intraveineuse, intramusculaire, intradermique, intrapéritonéale, intracérébrale, parentérale, etc... En terme de nature chimique, les **PA** plus particulièrement mais non limitativement concernés par l'invention sont hydrophiles ou amphipiles, par exemple, des protéines, des glycoprotéines, des peptides, des polysaccharides, des lipopolysaccharides, des polynucléotides et des molécules organiques.

La présente invention concerne, plus précisément, des suspensions colloïdales de Particules de Vectorisation, avantageusement de type submicronique, à base de blocs de polymères hydrophobes et de blocs de polyaminoacides hydrophiles, du type polyGlu.

La présente invention vise aussi bien des particules nues en tant que telles, que les systèmes de vectorisation de **PA,** constitués par les particules chargées par le (ou les) **PA.**

La présente invention a également trait à des solides pulvérulents comprenant ces **PV.** L'invention concerne, également, des procédés de préparation desdites suspensions colloïdales de particules, chargées en **PA.**

L'encapsulation de **PA** dans les **PV** a notamment, pour but de modifier leur durée d'action et/ou de les acheminer au lieu du traitement et/ou d'augmenter la biodisponibilité desdits **PA.** De nombreuses techniques d'encapsulation ont déjà été proposées. De telles techniques visent, d'une part, à permettre le transport du **PA** jusqu'à son site d'action thérapeutique, tout en le protégeant contre les agressions de l'organisme (hydrolyse, digestion enzymatique, etc.) et, d'autre part, à contrôler la libération du **PA** sur son site d'action, afin de maintenir la quantité disponible pour l'organisme au niveau désiré. Les **PA** concernés par ces avatars de transport et de séjour dans l'organisme sont, par exemple, des protéines mais peuvent être, également, des produits tout autres, des molécules organiques d'origine synthétique ou naturelle. La revue de M.J. HUMPHREY (Delivery system for peptide Drugs, éditée par S. DAVIS et L. ILLUM, Plenum Press, N.Y. 1986), fait état de la problématique concernant l'amélioration de la biodisponibilité des PA et l'intérêt des systèmes de vectorisation et de libération contrôlée.

Parmi tous les matériaux envisageables pour former des **PV,** les polymères sont de plus en plus utilisés, du fait de leurs propriétés intrinsèques. S'agissant du cahier des charges que l'on souhaite obtenir pour les **PV,** il est particulièrement exigeant et comprend, notamment, les spécifications suivantes.
1 La première spécification recherchée serait que les **PV** auraient avantage à pouvoir former, sans l'aide de solvant organique et/ou de tensioactif, une suspension aqueuse stable
2 Il est souhaitable que les **PV** et les systèmes **PV-PA** puissent être obtenus par un procédé non dénaturant pour le **PA.**
3 Une autre spécification recherchée serait que le polymère constituant les **PV** soit biocompatible, éliminable (par excrétion) et/ou biodégradable et, encore mieux, qu'il soit métabolisé en produits non toxiques pour l'organisme. En outre, il conviendrait que la biodégradation dans l'organisme soit d'une durée suffisamment courte.
4 Il serait également souhaitable que les **PV** aient une taille suffisamment faible pour pouvoir subir, en suspension dans un liquide, une filtration stérilisante par un filtre dont le diamètre des pores est inférieur ou égal à 0,2 µm.
5 Les **PV** devraient, avantageusement, permettre de contrôler la vitesse de libération du **PA.**
6 Une autre spécification importante serait que les systèmes **PV-PA** puissent constituer d'excellents médicaments injectables. Cette aptitude améliorée de l'administration par injection -e.g. intraveineuse, sous-cutanée ou intramusculaire - ou « injectabilité » se caractérise par :
   (i) un volume injecté réduit (pour une dose thérapeutique donnée),
   (ii) une viscosité faible.

   Ces deux propriétés sont satisfaites lorsque la dose thérapeutique de **PA** est associée à une quantité minimale de PV. En d'autres termes, les **PV** doivent avoir un fort taux de chargement en **PA.**
7 Le coût propre aux PV dans une préparation injectable doit être réduit et, là encore, il convient que les PV aient un fort taux de chargement en **PA**. En définitive, la faible taille et un fort taux de chargement sont des spécifications majeures recherchées pour les **PV.**
8 Il est également avantageux que le polymère, constitutif des **PV,** n'induise pas de réponse immunitaire.
9 Pour la famille de **PA** hydrophiles et amphiphiles, en particulier les protéines, il conviendrait d'avoir des **PV** qui soient adaptés à cette famille de **PA** en termes de facilité d'association et de libération et en termes de caractère non dénaturant.

Les propositions techniques antérieures, décrites infra, ont tenté de satisfaire l'ensemble de ces spécifications. A titre d'illustration, on citera les propositions antérieures (a) à (j) :
(a) Le brevet US-A-5 286 495 concerne un procédé d'encapsulation par vaporisation de protéines en phase aqueuse, à l'aide de matériaux ayant des charges opposées, à savoir : l'alginate (chargé négativement) et la polylysine (chargée positivement). Ce procédé de fabrication permet de produire des particules de taille supérieure à 35 µm.
(b) Par ailleurs, les techniques d'émulsion sont couramment utilisées pour préparer des microparticules chargées de **PA.** Par exemple, les demandes de brevets WO 91/06286, WO 91/06287 et WO 89/08449 divulguent de telles techniques d'émulsion dans lesquelles on a recours à des solvants organiques pour solubiliser des polymères, par exemple de type polylactique. Mais il s'est avéré que les solvants peuvent être dénaturants, notamment pour les **PA** peptidiques ou polypeptidiques.
(c) On connaît, également, des **PV** biocompatibles appelées protéinoïdes, décrites dès 1970 par X. FOX et K. DOSE dans « Molecular Evolution and the origin of Life », Ed. Marcel DEKKER Inc (1977). Ainsi, la demande de brevet WO 88/01213 propose un système à base d'un mélange de polypeptides synthétiques, dont la solubilité dépend du pH. Pour obtenir les microparticules matricielles selon cette invention, ils solubilisent le mélange de polypeptides, puis avec un changement de pH, ils provoquent la précipitation de particules protéinoïdes. Lorsque la précipitation s'effectue en présence d'un **PA,** celui-ci est encapsulé dans la particule.
(d) On mentionnera également, pour mémoire, le brevet US 4 351 337 qui relève d'un domaine différent de celui de la vectorisation de **PA** propre à l'invention. Ce brevet divulgue des implants massiques fixés et localisés à des endroits bien précis de l'organisme. Ces implants sont des tubes ou des capsules creuses de taille microscopique (160 µm et de longueur égale à 2 000 µm), constitués de copolymères de copoly(aminoacides) - e.g. poly(acide glutamique-leucine) ou poly(benzylglutamate-leucine) - obtenus par copolymérisation de monomères de N-carboxyanhydrides d'aminoacides (NCA). L'inclusion d'un **PA** s'opère par une technique d'évaporation de solvant d'un mélange de polymère et de **PA.** Le brevet US 4 450 150 appartient à la même famille que le brevet US 4 351 337 étudié ci-dessus et a essentiellement le même objet. Les PAA constitutifs sont des poly(acide glutamique-éthylglutamate).
(e) La demande de brevet PCT WO 97/02810 divulgue une composition pour la libération contrôlée de principes actifs, comprenant une pluralité de particules lamellaires d'un polymère biodégradable, au moins en partie cristallin (polymère d'acide lactique) et d'un **PA** absorbé sur lesdites particules. Dans ce cas, la libération du principe actif s'opère par désorption.
(f) La demande de brevet PCT WO 96/29991 a pour objet des particules de polyaminoacides utiles pour la vectorisation de **PA** dont notamment des **PA** hydrophiles tels que l'insuline. Ces particules ont une taille comprise entre 10 et 500 nm. Les particules selon le WO 96/29991 se forment spontanément par mise en contact de PAA avec une solution aqueuse. Les PAA comprennent des monomères aminoacides neutres et hydrophobes AAO et des monomères ionisables et hydrophiles AAI.
   Ces particules peuvent être chargées en insuline, au mieux à hauteur de 6,5 % en poids sec d'insuline par rapport à la masse de PAA. Le taux de chargement, Ta, est mesuré selon un mode opératoire Ma décrit plus loin.
(g) La demande de brevet EP 0 583 955 (US-B 5 449 513) divulgue des micelles polymère capables de piéger physiquement des **PA** hydrophobes. Ces micelles sont constituées par des copolymères bloc : PEG/polyAANO, AANO = Amino-Acide Neutre hydrophObe. L'AANO peut être : Leu, Val, Phe, Bz-O-Glu, Bz-O-Asp, ce dernier étant préféré. Les principes actifs PA hydrophobes piégés dans ces micelles PEG/polyAANO sont e.g. : Adriamycine, indométhacine, daunomycine, methotrexate, mitomycine.
(h) Le brevet US N° 5 514 380 divulgue un copolymère comprenant un bloc de polymère d'acide lactique et un bloc de polyoxyde d'éthylène (PEG), utile comme matrice pour la libération de médicaments. Il n'est pas question de microparticules réalisées à partir de ce copolymère.
(i) On connaît par ailleurs de nombreuses publications qui décrivent des particules à base de copolymères PEG/polymère d'acide lactique (**PAL**), pour la libération prolongée de principes actifs, dont notamment :
   - Biomatérials 17 (1996) 1575-1581, Vittaz et al,
   - Polym.Adv.Technol. 10, 647-654 (1999), Kinn et al.

   Dans ces particules copoly(PEG)(PAL), le **PA** est encapsulé physiquement dans le coeur de **PAL** par codissolution dans un solvant organique du **PA** et du copoly-(PEG)(PAL). Il en résulte que les **PA** formés par des protéines pourraient difficilement être encapsulés dans ces particules copoly(PEG)(PAL) car les risques de dénaturation du PA sont importants.
(j) L'article Biomaterials 19(1998) 1501-1505 / K.E. GONSALVES *et al* décrit des microparticules (diamètre = 200 nm) de copolymère poly(L-lactique)(Asp) et de copolymère poly(L-lactique)(Ser). Ces particules copoly(PAL)(PAA) - PAA = PolyAminoAcide- sont obtenues sous forme d'émulsion par agitation mécanique d'une solution aqueuse d'Alcool Poly Vinylique (APV) stabilisante (tensioactif) et une solution organique (CH₂Cl₂) de copoly(PAL)(PAA). Ces particules sphériques creuses sont stabilisées grâce au tensioactif APV, qui forme une couche externe, la couche interne étant constituée du copoly(PAL)(PAA). Ces particules nécessitent pour exister l'usage du tensioactif stabilisant APV. Il n'est pas question d'ionisation au moins partielle du PAA. En outre, les auteurs supputent que ces particules copoly (PAL) (PAA) pourraient être utilisées pour la libération controlée de médicaments. Cette supputation n'est étayée par aucune expérience technique. Cet article ne divulgue pas de suspension colloïdale stable comprenant ces microparticules, et encore moins une quelconque aptitude de ces dernières à s'associer en suspension colloïdale à l'état non dissous, avec au moins un principe actif.

Il ressort de ce qui précède que les propositions techniques antérieures sus décrites, satisfont incomplètement aux spécifications du cahier des charges indiqué supra, et en particulier pour ce qui concerne l'association des particules à des principes actifs (en particulier les protéines) ainsi que l'aptitude de ces particules chargées en **PA** à libérer ces derniers in vivo sans qu'ils n'aient été altérés par la vectorisation.

Dans cet état de fait, un objectif essentiel est de pouvoir fournir de nouvelles **PV** qui forment spontanément, et sans l'aide de tensioactifs, des suspensions aqueuses stables (aux pH physiologiques) de **PV** et adaptées à la vectorisation de **PA** (notamment des **PA** sensibles tels que des protéines).

Un autre objectif essentiel de la présente invention est de fournir de nouvelles **PV** en suspension aqueuse colloïdale stable ou sous forme pulvérulente et à base de poly(aminoacides) (PAA), ces nouvelles **PV** se devant de satisfaire au mieux aux spécifications 1 à 9 du cahier des charges susvisé.

Un autre objectif essentiel de l'invention est de fournir une suspension nouvelle de **PV** dont on maîtrise parfaitement les caractéristiques, notamment en termes du taux de chargement en **PA** et en termes de contrôle de cinétique de libération du **PA.**

Un autre objectif essentiel de l'invention est de fournir des suspensions médicamenteuses injectables. Les spécifications, requises pour de telles suspensions, sont un faible volume d'injection et une faible viscosité. Il importe que la masse de particules colloïdales par dose d'injection soit la plus faible possible et ce sans limiter la quantité du principe actif **PA** transporté par ces particules, afin de ne pas nuire à l'efficacité thérapeutique.

Un autre objectif essentiel de l'invention est de fournir une suspension colloïdale aqueuse ou un solide pulvérulent comprenant des particules de vectorisation de principes actifs satisfaisant aux spécifications visées ci-dessus et qui constitue une forme galénique appropriée et convenable pour une administration, par exemple orale, à l'homme ou l'animal.

Un autre objectif essentiel de l'invention est de fournir une suspension colloïdale comprenant des particules de vectorisation de principes actifs filtrables sur des filtres de 0,2 µm à des fins de stérilisation.

Un autre objectif essentiel de l'invention est de proposer un procédé de préparation de particules (sèches ou en suspension dans un liquide) de blocs PAA hydrophobe/polymère hydrophile utiles, notamment, comme vecteurs de principes actifs (notamment protéines telles que l'insuline, l'IFN, l'IL-2, le facteur VIII, l'EPO, etc), ledit procédé se devant d'être, plus simple à mettre en oeuvre, non dénaturant pour les principes actifs et devant en outre toujours permettre une maîtrise fine de la granulométrie moyenne des particules obtenues.

Un autre objectif essentiel de l'invention est l'utilisation des susdites particules en suspension aqueuse ou sous forme solide pour la préparation de médicaments (e.g. vaccins), en particulier pour administration notamment orale, nasale, vaginale, oculaire, sous-cutanée, intraveineuse, intramusculaire, intradermique, intrapéritonéale, intracérébrale ou parentérale, les principes actifs hydrophiles de ces médicaments pouvant être, notamment, des protéines, des glycoprotéines, des peptides, des polysaccharides, des lipopolysaccharides, des oligonucléotides et des polynucléotides.

Un autre objectif de la présente invention est de fournir un médicament, du type système à libération prolongée de principes actifs, qui soit aisé et économique à produire et qui soit, en outre, biocompatible et apte à assurer un très haut niveau de biodisponibilité du **PA.**

Les objectifs relatifs aux produits (parmi d'autres) sont atteints par la présente invention qui concerne, tout d'abord, une suspension colloïdale de particules submicroniques susceptibles d'être utilisées, notamment pour la vectorisation de **PA,** ces particules étant des arrangements supramoléculaires individualisés, à base d'un copolymère amphiphile comprenant :
► au moins un bloc de polyaminoacide(s) (PAA) linéaire(s), hydrophile(s) à enchaînements α-peptidiques, les aminoacides hydrophiles AAI constitutifs de ce bloc PAA étant identiques ou différents entre eux ;
► et au moins un bloc d'au moins un polymère hydrophobe, formé par un Polymère d'Acide α-HydroxyCarboxylique (PAHC) - de préférence Polymère d'Acide Lactique (PAL)ou Polymère d'Acide Glycolique (PAG)-
caractérisée en ce que :
- elle peut être obtenue spontanément en l'absence de tensioactif, par mise en présence de copolymère amphiphile avec un liquide non-solvant du copolymère amphophile
- elle est stable même en l'absence de tensioactif ;
- les AAI du copolymère sont au moins partiellement sous forme ionisée ;
- les particules sont aptes à s'associer en suspension colloïdale à l'état non dissous, avec au moins un **PA** et à libérer celui-ci, notamment in vivo, de manière prolongée et/ou retardée.

L'un des fondements inventifs de ces nouvelles particules de vectorisation **PV,** en suspension aqueuse colloïdale stable aux pH physiologiques ou à l'état de solide pulvérulent, tient à la sélection originale d'un copolymère bloc (polymère d'acide(s) α-hydroxycarboxylique(s) hydrophobe) / (polyaminoacide hydrophile) permettant d'obtenir des particules de taille submicronique, qui forment une suspension colloïdale (de préférence aqueuse) stable à tous pH physiologiques, en l'absence de tensioactifs qui soient adaptés à tous pH.

Le fait que ces microparticules (PAHC)(PAA) aient au moins une partie de leurs AAI sous forme ionisée en suspension, constitue également une caractéristique innovante.

Un autre avantage remarquable de ces particules submicroniques tient à leur capacité à permettre l'adsorption à leur surface de **PA,** en suspension colloïdale à l'état non dissous, et donc en l'absence de tout solvant organique ou tensioactif agressif. Ce type d'association est à distinguer des processus d'encapsulation physique de **PA** en solution, dans des coeurs de microparticules. De telles conditions d'encapsulation sont dénaturantes pour certains **PA.** Il n'en est rien s'agissant des microparticules selon l'invention.

En outre, il est particulièrement surprenant et inattendu que les particules à base de copolymère bloc amphiphile *poly(AAI)*/*(polylactide et*/*ou glycolide et*/*ou caprolactone),* puissent s'associer et libérer in vivo des **PA**, en particulier des protéines.

La structure des copolymères bloc PAHC/PolyAAI et la nature des acides aminés AAI, sont choisies de telle façon que :
les chaînes de polymères se structurent spontanément sous forme de particules (**PV**) de petite taille;
**•** les particules forment une suspension colloïdale stable dans l'eau et en milieu physiologique (pH=6-8) ;
• les **PV** s'associent à l'état colloïdal non dissous avec des protéines ou autres **PA** en milieu aqueux, par un mécanisme spontané et non dénaturant pour le **PA;**
• les PV libèrent les **PA** en milieu physiologique et, plus précisément, in vivo ; la cinétique de libération est fonction de la nature du copolymère PAHC/Poly AAI précurseur des **PV.**

Ainsi, en jouant sur la structure particulière du copolymère, on peut contrôler les phénomènes d'association et de libération du **PA** sur le plan cinétique et quantitatif.

De préférence, la suspension est caractérisée en ce qu'elle est obtenue par mise en solution du copolymère amphiphile dans un solvant organique et mise en présence de cette solution avec un liquide aqueux.

Pour définir un peu plus les copolymères constitutifs des particules, on peut indiquer qu'ils sont du type séquentiel alterné (blocs).

Ainsi, selon une forme préférée de réalisation des **PV** selon l'invention :
- les AAI sont des acides aminés hydrophiles AAI ;
- le rapport AHC/AAI est supérieur à 0,1 ;
- la longueur absolue du bloc PAHC est supérieure à 2 monomères, de préférence supérieure à 10 monomères; et plus préférentiellement comprise entre 20 et 100 monomères.

Dans la présente demande, on entend par AHC, un monomère constitutif du PAHC.

Avantageusement, le ou les blocs PAA à base d'AAI en comprennent au moins 5, de préférence au moins 20, et plus préférentiellement encore entre 30 et 100.

De manière plus préférée encore, les particules sont des "diblocs" de PAHC/AAI.

Le(ou les)AAI est(sont) choisi(s) parmi des aminoacides à chaîne latérale ionisable, les aminoacides naturels Glu et Asp sous forme carboxylique et/ou sous forme de sels étant particulièrement préférés.

Les PAA blocs constitutifs de particules ont, par exemple, des degrés de polymérisation DP compris entre 30 et 600, de préférence entre 50 et 200 et, plus préférentiellement encore, entre 60 et 150.

La présente invention vise non seulement des suspensions de particules nues, telles que définies ci-dessus, mais également des particules comprenant au moins un principe actif **PA**. De préférence, la suspension selon l'invention est aqueuse et stable. Ces particules, chargées ou non en **PA,** sont, avantageusement, sous forme dispersée dans un liquide (suspension), de préférence aqueux, mais peuvent également être à l'état de solide pulvérulent, obtenu à partir de la suspension de **PV** telle que définie ci-dessus.

D'où il s'ensuit que l'invention concerne, outre une suspension colloïdale (de préférence aqueuse) de **PV,** un solide pulvérulent comprenant des PV et obtenu à partir de la suspension selon l'invention.

Un autre objet essentiel de l'invention se rapporte à la préparation des particules sélectionnées (telles que décrites ci-avant), aussi bien sous forme de suspension colloïdale que sous forme de solide pulvérulent. Le procédé de préparation considéré consiste, essentiellement, à synthétiser des copolymères PAHC/polyAAI précurseur et à les transformer en particules structurées.

Plus précisément, il s'agit, tout d'abord, d'un procédé de préparation du solide pulvérulent susvisé et formé par des particules submicroniques susceptibles d'être utilisées, notamment pour la vectorisation de principe(s) actif(s) (**PA**), ces particules étant des arrangements supramoléculaires individualisés :
- à base d'un copolymère amphiphile comprenant :
   ✔ au moins un bloc de polyaminoacide(s) (PAA) linéaire(s), hydrophile(s) à enchaînements α-peptidiques, les aminoacides hydrophiles AAI constitutifs de ce bloc PAA étant identiques ou différents entre eux ;
   ✔ et au moins un bloc de polymère(s) hydrophobe(s) à base de Polymère(s) d'Acide(s) α-HydroxyCarboxylique(s) (PAHC) - de préférence Polymère(s) d'Acide Lactique(PAL)ou Polymère(s) d'Acide Glycolique (PAG) - ;
- aptes à former une suspension colloïdale, même en l'absence de tensioactifs ;
- aptes à s'associer en suspension colloïdale à l'état non dissous, avec au moins un **PA** et à libérer celui-ci, notamment in vivo, de manière prolongée et/ou retardée.

Ce procédé est caractérisé en ce que :
- 1)- on met en oeuvre ou on prépare par polymérisation de monomères d'acide(s) α-hydroxycarboxylique(s)-de préférence acide lactique ou glycolique- au moins un bloc PAHC (de préférence de PAL ou PAG); ce bloc PAHC étant fonctionnalisé (avantageusement à au moins l'une de ses extrémités) par au moins un groupement réactif protecteur, de préférence choisi dans le groupe comprenant la BOC-éthanolamine, BOC-aminopropanol, (BOC = ButOxyCarbonyle) ;
- 2)- on déprotège le bloc PAHC de l'étape -1)- ;
- 3)- on réalise une copolymérisation de monomères formés par des anhydrides de N-CarboxyAminoacides (NCA) d'amino-acides hydrophiles AAI et/ou par des anhydrides de N-CarboxyAminoacides (NCA) précurseurs d'amino-acides hydrophiles AAI et porteurs de groupements protecteurs, en présence d'au moins un solvant organique, de préférence choisi dans le groupe comprenant : la N-MéthylPyrrolidone (NMP), le DiMéthylFormamide (DMF), le DiMéthylsulfOxyde (DMSO), le DiMéthylAcétamide (DMAc), la pyrrolidone et le dichlorométhane ; ce dernier étant plus particulièrement préféré ;
- 4)- on ajoute le bloc PAHC déprotégé de l'étape -2)- au milieu de polymérisation du bloc de poly-AAI, avant, pendant ou après la polymérisation;
- 5)- éventuellement on déprotège les précurseurs d'amino-acides hydrophiles AAI pour obtenir un ou plusieurs blocs polyAAI ;
- 6)- on précipite le copolymère bloc PAHC-polyAAI obtenu à l'issue des étapes précédentes ;
- 7)- on met en solution le précipité de copolymère bloc PAHC-polyAAI obtenu à l'étape -6)- et on met en présence cette solution avec un liquide contenant au moins un non-solvant du copolymère bloc PAHC-polyAAI, de préférence l'eau, ce liquide ayant un pH choisi de telle sorte que les AAI du copolymère bloc PAHC-polyAAI soient au moins en partie ionisés ;
- 8)- éventuellement on associe au moins un principe actif **PA** hydrophile avec les particules ;
- 9)- éventuellement on purifie la suspension de l'étape -7)- ;
- 10)- éventuellement on concentre la suspension de l'étape -7)- ;
- 11)- on élimine le milieu liquide pour recueillir le solide pulvérulent comprenant les particules.

Avantageusement, les PAHC de l'étape -1)- sont obtenus de manière connue en soi par polymérisation de lactide, de glycolide, ou de caprolactone, ou bien encore sont des produits commerciaux disponibles (polytactide, polylactide/glycolide, polycaprolactone e.g.).

Des procédés d'obtention de ces PAHC sont décrits par exemple dans les brevets suivants : US 4 835 293, US 5 023 349, FR 2 692 263.

La déprotection selon l'étape -2)- s'effectue de manière connue en soi, par exemple par hydrolyse acide (e.g. acide trifluoroacétique).

La troisième étape du procèdé s'inspire des techniques connues de polymérisation d'anhydrides de N-carboxy-α-aminoacides (NCA), décrites, par exemple, dans l'article « Biopolymers, 15, 1869 (1976) » et dans l'ouvrage de H.R. KRICHELDORF "α-Aminoacid-N-carboxy Anhydride and Related Heterocycles » Springer Verlag (1987)".

De préférence, le (ou les) bloc(s) PAHC fonctionnalisé(s) est (sont) introduit(s) avant et/ou au début de la polymérisation selon l'étape -3)-, qui se déroule de préférence à une température comprise entre 20 et 120°C à pression atmosphérique normale.

Avantageusement, cette étape -3)- est réalisée en présence d'au moins un co-solvant sélectionné parmi les solvants aprotiques (de préférence le dioxanne-1,4) et/ou les solvants protiques (de préférence la pyrrolidone) et/ou l'eau et/ou les alcools, le méthanol étant particulièrement préféré.

De manière plus préférée encore, les NCA-AAI sont des NCA d'acide glutamique ou aspartique O-alkylé ou O-arylé, par exemple des NCA-Glu-O-Me, NCA-Glu-O-Et ou NCA-Glu-O-Bz (Me = méthyle / Et = éthyle / Bz = benzyle).

D'autres paramètres expérimentaux, comme :
o la concentration en NCA et/ou en polymère bloc PAHC dans le solvant organique (de préférence le dichlorométhane) ;
o et/ou la concentration ou la nature de l'éventuel cosolvant, lors de la synthèse ;
o la température du mélange réactionnel ;
o le mode d'ajout du polymère hydrophile ;
o l'emploi de pression réduite;
o la durée de la réaction, etc... ;
sont ajustés selon les effets désirés et bien connus de l'homme de l'art.

Suivant une variante dans laquelle le procédé est interrompu à l'issue d'une étape - 5bis)- succédant à l'issue de l'étape -5)-, on précipite - de préférence dans l'eau - le copolymère PAHC-polyAAI obtenu et on recueille ce précipité. Cette variante correspond à un mode discontinu de préparation de particules, dans lequel on isole le copolymère PAHC-polyAAI sous forme de précipité formant un produit intermédiaire stable. Ce précipité peut être, par exemple, filtré, lavé et séché.

Pour effectuer l'association de l'étape -8)- d'un ou plusieurs **PA** aux particules, il est possible de mettre en oeuvre plusieurs méthodes conformément à l'invention.

Des exemples, non limitatifs, de ces méthodes sont énumérés ci-après.

Selon une première méthode, on effectue l'association de **PA** aux particules par mise en présence d'une phase liquide (aqueuse ou non) contenant le **PA** avec la suspension colloïdale de particules.

Selon une deuxième méthode, on effectue l'association du **PA** aux particules par mise en présence d'un **PA** à l'état solide avec la suspension colloïdale de particules. Le **PA** solide peut être, par exemple, sous forme de lyophilisat, de précipité, de poudre ou autre.

Selon une troisième méthode, on met en présence le solide pulvérulent (polylactide/polyAAI), tel que décrit supra en tant que produit et par ses caractéristiques d'obtention, avec une phase liquide (aqueuse ou non) contenant le **PA.**

Selon une quatrième méthode, on met en présence le solide pulvérulent, tel que décrit supra en tant que produit et par ses caractéristiques d'obtention, avec le PA sous forme solide. On disperse ensuite ce mélange de solides dans une phase liquide, de préférence une solution aqueuse.

Dans toutes ces méthodes, le **PA** utilisé peut être sous forme pure ou préformulée.

Conformément à l'étape facultative -9)-, on élimine les impuretés (sels) ainsi que le solvant, par tout traitement de séparation physique approprié, par exemple par diafiltration (dialyse), filtration, modification du pH, chromatographie...

Cela conduit à une suspension (de préférence aqueuse) de particules structurées qui peut être concentrée [étape -10)-], par exemple par distillation ou tout autre moyen physique convenable : ultrafiltration, centrifugation.

Pour séparer, à l'étape -11)-, les particules de leur milieu liquide de suspension, on élimine, éventuellement, la phase aqueuse, par exemple par séchage (e.g. à l'étuve), par lyophilisation ou tout autre moyen physique convenable : ultrafiltration, centrifugation. On récupère, à l'issue de cette étape -11)-, un solide pulvérulent de couleur blanche.

Il est à noter que la mise en oeuvre des étapes -1)-, -2)-, -3)-, -4)-, -5)-, -6)-, -7)-, et éventuellement -8)- du procédé ci-dessus correspondent à une préparation d'une suspension colloïdale de particules submicroniques et à fort taux de chargement en **PA.**

Lors de cette préparation de suspension colloïdale, les copolymères amphiphiles polylactide et/ou polyglycolide et/ou polycaprolactone-poly(AAI) de l'étape -6)- sont placés dans un milieu aqueux dans lequel au moins une partie des PAHC est soluble et au moins une partie des AANO est insoluble. Les copolymères PAHC/polyAAI existent sous forme de nanoparticules dans ce milieu aqueux.

Une alternative pour préparer la suspension de **PV** selon l'invention consiste à mettre en présence le solide pulvérulent, tel que décrit ci-dessus en tant que produit et par son procédé d'obtention, avec un milieu aqueux, non-solvant du copolymére amphiphile.

Compte tenu de la taille nanométrique des particules, la suspension peut être filtrée sur des filtres de stérilisation, ce qui permet d'obtenir, aisément et à moindre coût, des liquides médicamenteux injectables stériles. Le fait de pouvoir, grâce à l'invention, faire subir à la suspension de particules une filtration stérilisante est un atout important.

La présente invention vise, également, de nouveaux produits intermédiaires du procédé décrit ci-dessus, caractérisés en ce qu'ils sont constitués par des copolymères PAHC-polyAAI précurseurs de particules.

Selon un autre de ses aspects, l'invention concerne une suspension et/ou un solide pulvérulent, tels que définis ci-dessus et/ou tels qu'obtenus par le procédé présenté supra, cette suspension et ce solide comprenant au moins un principe actif hydrophile, choisi, de préférence, parmi :
- les vaccins ;
- les protéines et/ou les peptides, parmi lesquels les plus préférentiellement retenus sont : les hémoglobines, les cytochromes, les albumines, les interférons, les cytokines, les antigènes, les anticorps, l'érythropoïétine, l'insuline, les hormones de croissance, les facteurs VIII et IX, les interleukines ou leurs mélanges, les facteurs stimulants de l'hématopoïèse ;
- les polysaccharides, l'héparine étant plus particulièrement sélectionnée ;
- les acides nucléiques et, préférablement, les oligonucléotides d'ARN et/ou d'ADN,
- des molécules non peptido-protéiques appartenant à diverses classes de chimiothérapie anticancéreuses et, en particulier, les anthracyclines et les taxoïdes ;
- et leurs mélanges.

Enfin, l'invention concerne une spécialité pharmaceutique, nutritionnelle, phytosanitaire ou cosmétique, caractérisée en ce qu'elle comporte une suspension et/ou du solide pulvérulent chargé(s) en **PA** hydrophile et te(s) que défini(s) ci-dessus.

Selon un autre de ses objets, l'invention vise, également, l'utilisation de ces PV (en suspension ou sous forme solide) chargées en **PA,** pour la fabrication de médicaments du type systèmes à libération contrôlée de **PA.**

Il peut s'agir, par exemple de ceux administrables, de préférence par voie orale, nasale, vaginale, oculaire, sous-cutanée, intraveineuse, intramusculaire, intradermique, intrapéritonéale, intracérébrale ou parentérale.

Les applications cosmétiques envisageables sont, par exemple, les compositions comprenant un **PA** associé aux **PV** selon l'invention et applicables par voie transdermique.

Enfin, l'invention concerne une spécialité pharmaceutique, nutritionnelle, phytosanitaire ou cosmétique, caractérisée en ce qu'elle comporte une suspension et/ou du solide pulvérulent chargé(s) en **PA** et te(s) que défine(s) ci-dessus.

Selon un autre de ses objets, l'invention vise, également, l'utilisation de ces **PV** (en suspension ou sous forme solide) chargées en **PA,** pour la fabrication de médicaments du type systèmes à libération contrôlée de **PA.**

Dans le cas de médicaments, il peut s'agir, par exemple de ceux administrables, de préférence par voie orale, nasale, vaginale, oculaire, sous-cutanée, intraveineuse, intramusculaire, intradermique, intrapéritonéale, intracérébrale ou parentérale.

Les applications cosmétiques envisageables sont, par exemple, les compositions comprenant un **PA** associé aux **PV** selon l'invention et applicables par voie transdermique.

Les produits phytosanitaires concernés peuvent être, par exemple, des herbicides, des pesticides, des insecticides, des fongicides, etc...

Les exemples qui suivent permettront de mieux comprendre l'invention dans ses différents aspects produit/procédé/application. Ces exemples illustrent la préparation de particules de polylactide/PAAI chargés ou non en principes actifs, de même qu'ils présentent les caractéristiques de structure et les propriétés de ces particules.

### DESCRIPTION DES FIGURES

**Figure 1** **:**
   Isotherme d'adsorption de l'insuline (9, 3mg/ml) sur la dispersion de nanoparticules de l'exemple 8.
**Figure 2** **:**
   Profils d'insulinémie et de glycémie chez le cochon sain après administration d'une dose de 0,6 IU/kg d'insuline adsorbée sur les particules de l'exemple 7.

### EXEMPLES :

La synthèse des copolymères blocs se déroule en quatre grandes étapes :
   1. polymérisation du lactide avec un amorceur bifonctionnel protégé ;
   2. déprotection de l'amorceur lié au polymère;
   3. polymérisation du second monomère sur la fonction déprotégée de l'amorceur ;
   4. déprotection des groupements protecteurs sur le second monomère.

### Exemple 1 : poly(acide lactique)₂₀-bloc-(acide glutamique)₅₀

**1.1 BOC-aminopropyl-poly(acide lactique)₂₀ :** L-lactide (5 g, 34,70 mmol, Aldrich 16101-127) et toluène distillé (27ml) sont introduits dans un ballon sec et sous azote. On chauffe une heure à 80°C. Dans un second ballon on prépare un mélange de t-butoxycarbonyl-aminopropanol (0,58 g, 3,30 mmol, Fluka 381029/1) et de toluène fraîchement distillé (23ml), qui est refroidi à -10°C. Après l'ajout du diéthylzinc (1,5 ml, 1,65 mmol, 1,1 M dans le toluène Aldrich 72560-099) au BOC-aminopropanol, on permet à ce milieu réactionnel de devenir à température ambiante, puis on l'ajoute sur le monomère lactide pour initier la polymérisation. La polymérisation est terminée par un ajout de 4ml d'acide acétique en solution dans le toluène (10%). Le milieu réactionnel est alors concentré à l'évaporateur rotatif et précipité dans un large excès de méthanol. Le polymère est récupéré par filtration puis séché sous vide. Rendement : 98 %. *Caractérisations* : Tg : 30-37°C. RMN ¹H (CDCl₃) : 8 = 1,4 ppm (s, 9H, CCH₃), 1,55 ppm (d, 6H, ³J=7,1Hz, CHCH₃), 1,85 ppm (q, 2H, ³J=6,3Hz, CH₂CH₂CH₂), 3,1 ppm (q, 2H, ³J=6,2Hz, CH₂NH), 4,15 ppm (t, 2H, ³J=6,0Hz, CH₂O), 4,35 ppm (q, 1H, ³J=6,9Hz, CH₃CHOH), 4,8 ppm (m, 1H, NH), 5,15 ppm (q, 2H, ³J=7,1Hz, CHCH₃). RMN ¹³C (CDCl₃) : δ = 17 ppm (2C, CHCH₃), 20,7 ppm (1C, CH₃CHOH), 28,7 ppm (3C, CH₃C), 29,5 ppm (CH₂CH₂CH₂), 37,5 ppm (CH₂NH), 63,5 ppm (CH₂O), 67 ppm (CH₃CHOH), 69,5 ppm (2C, CH), 156 ppm (NHC=O), 170 ppm (CHC(O)O).
**1.2 aminopropyl-poly(acide lactique)₂₀:** Du polylactide (4g, 2,65mmol) et le dichlorométhane distillé (45ml) sont introduits sous courant d'azote dans un ballon. On introduit l'acide trifluoroacétique (8ml, 0,1 mol, Sigma 19H3648) et on place la solution sous agitation à température ambiante pendant une demi-heure, jusqu'à ce que le dégagement de CO₂ soit terminé. On évapore les solvants du milieu réactionnel à l'évaporateur rotatif. On ajoute 40ml de dichlorométhane et la solution est lavée deux fois par 40ml de NaHCO₃ en solution aqueuse (5%) puis deux fois par 40ml d'eau distillée jusqu'à ce que le pH des eaux de lavage soit neutre. La phase organique est séchée sur MgSO₄ puis filtrée. Le solvant est évaporé à l'évaporateur rotatif, puis le polymère est séché sous vide. Rendement : 95%. *Caractérisations :* RMN ¹H (CDCl₃) : δ = 1,55 ppm (d, 6H, ³J=7,1Hz, CH3), 1,85 ppm (q, 2H, ³j=6,3Hz, CH₂CH₂CH₂), 2,8 ppm (q, 2H, ³J=6,2Hz, CH₂NH₂), 4,15 ppm (t, 2H, ³J=6,0Hz, CH₂O), 4,35 ppm (q, 1H, ³J=6,9Hz, CH₃CHOH), 5,15 ppm (q, 2H, ³J=7,1Hz, CH).
**1.3 poly(benzyl glutamate)₅₀-propyl-poly(acide lactique)₂₀:** Du N-carboxyanhydride de L-glutamate de benzyle (8,68g, 33,0 mmol) est introduit dans un ballon. Le polylactide déprotégé (1g, 0,66 mmol) est solubilisé dans du dichlorométhane fraîchement distillé (40ml) puis introduit par canule. Le milieu réactionnel est placé sous agitation magnétique pendant trois heures à température ambiante. Le solvant est évaporé au rotavapor puis le polymère est séché sous vide primaire. Rendement: 85%. *Caractérisations :* RMN ¹H (TFA d) : δ = 1,55 ppm (m, 6H, CH₃), 1,95 ppm (m, 2H, CH₂CH₂C=O), 2,35 ppm (m, 2H, CH₂CH₂C=O), 4,60 ppm (m, 1H, O=CCHNH), 5,0ppm (m, 2H, CH), 5,25 ppm (m, 2H, CH₂Ph), 7,10 ppm (m, 5H, Ph). RMN ¹³C (DMSO d6) : δ = 17 ppm (CH₃), 27 ppm (CH₂CH₂COO), 30 ppm (CH₂CH₂COO), 52 ppm (O=CCHNH), 66 ppm (CH₂Ph), 128-136 ppm (Ph), 168-170 ppm (OC=O), 172 ppm (NC=O).
**1.4 poly(acide glutamique)₅₀-propyl-poly(acide lactique)₂₀:** Le copolymère (5g, 20mmol d'ester de benzyle) est introduit dans un ballon et solubilisé dans l'acide trifluoroacétique (44ml, 0,57mol) à 10°C. On introduit l'acide méthane sulfonique (44ml, 0,68mol) et l'anisole (11ml, 0,10mol) sous courant d'azote et on laisse le milieu réactionnel trois heures sous agitation. Le polymère est précipité dans un large excès d'éther éthylique froid, récupéré par filtration, lavé avec de l'éther éthylique et séché sous vide. Rendement : 99%. *Caractérisations :* RMN ¹H (TFA d) : δ = 1,55 ppm (m, 6H, CH₃), 1,95 ppm (m, 2H, CH₂CH₂C=O), 2,35 ppm (m, 2H, CH₂CH₂C=O), 4,60 ppm (m, 1H, O=CCHNH), 5,0ppm (m, 2H, CH). RMN ¹³C (DMSO d6) _{:} δ = 17 ppm (CH3), 27 ppm (CH₂CH₂COOH), 30 ppm (CH₂CH₂COOH), 52 ppm (OCCHNH), 69 ppm (CH), 168-170 ppm (O=CO), 172 ppm (O=CNH).

### Exemple 2 : poly(acide lactique)₃₀-bloc-(acide glutamique)₈₀

**2.1 BOC-aminopropyl-poly(acide lactique)₃₀ :** Le L-lactide (5g, 34,70mmol, Aldrich 16101-127) est introduit en boîte à gants sous atmosphère d'azote dans un ballon préalablement flammé. Le toluène, fraîchement distillé (27ml) est introduit par canule dans le ballon que l'on place sous agitation magnétique pendant une heure à 80°C. Le ter-butoxycarbonyl aminopropanol (0,40g, 2,30mmol, Fluka 381029/1) et le toluène fraîchement distillé (23ml) sont introduits dans un ballon préalablement flammé et placé sous agitation magnétique dans un bain à -10°C. La solution de diéthylzinc dans le toluène (1,0ml, 1,15mmol, 1,1M, Aldrich 72560-099) est introduite goutte-à-goutte dans cette solution. Le milieu réactionnel est alors laissé sous agitation magnétique à température ambiante. Au bout d'une heure on introduit la solution de L-lactide sous courant d'azote dans le milieu réactionnel que l'on place alors à 80°C sous agitation pendant une heure. La polymérisation est terminée par un ajout de 4ml d'acide acétique en solution dans le toluène (10%). Le milieu réactionnel est alors concentré à l'évaporateur rotatif et précipité dans un large excès de méthanol froid. Le polymère précipité est récupéré par filtration puis séché sous vide primaire. Rendement : 98%. *Caractérisations* : Tg : 30-37°C. RMN ¹H (CDCl₃) : δ = 1,4 ppm (s, 9H, CCH₃), 1,55 ppm (d, 6H, ³J=7,1Hz, CHCH₃), 1,85 ppm (q, 2H, ³J=6,3Hz, CH₂CH₂CH₂), 3,1 ppm (q, 2H, ³J=6,2Hz, CH₂NH), 4,15 ppm (t, 2H, ³J=6,0Hz, CH₂O), 4,35 ppm (q, 1H, ³J=6,9Hz, CH₃CHOH), 4,8 ppm (m, 1H, NH), 5,15 ppm (q, 2H, ³J=7,1Hz, CHCH₃). RMN ¹³C (CDCl₃) : δ = 17 ppm (2C, CHCH₃), 20,7 ppm (1C, CH₃CHOH), 28,7 ppm (3C, CH₃C), 29,5 ppm (CH₂CH₂CH₂), 37,5 ppm (CH₂NH), 63,5 ppm (CH₂O), 67 ppm (CH₃CHOH), 69,5 ppm (2C, CH), 156 ppm (NHC=O), 170 ppm (CHC(O)O).
**2.2 aminopropyl-poly(acide lactique)₃₀** : Le polylactide (4g, 1,85mmol) et le dichlorométhane fraîchement distillé (45ml) sont introduits sous courant d'azote dans un ballon préalablement flammé et relié à un bulleur. On introduit l'acide trifluoroacétique (8ml, 0,1mol, Sigma 19H3648) et on place la solution sous agitation à température ambiante pendant une demi-heure, jusqu'à ce que le dégagement de CO₂ soit terminé. On évapore les solvants du milieu réactionnel à l'évaporateur rotatif. On solubilise le polylactide dans 40ml de dichlorométhane. Cette phase organique est lavée deux fois par 40ml de NaHCO₃ en solution aqueuse (5%) puis deux fois par 40ml d'eau distillée jusqu'à ce que le pH des eaux de lavage soit neutre. La phase organique est alors séchée sur MgSO₄ puis filtrée. Le solvant est évaporé à l'évaporateur rotatif, puis le polymère est séché sous vide primaire. Rendement : 95%. *Caractérisations* : RMN ¹H (CDCl₃) : δ = 1,55 ppm (d, 6H, ³J=7;1Hz, CH3), 1,85 ppm (q, 2H, ³J=6,3Hz, CH₂CH₂CH₂), 2,8 ppm (q, 2H, ³J=6,2Hz, CH₂NH₂), 4,15 ppm (t, 2H, ³J=6,0Hz, CH₂O), 4,35 ppm (q, 1H, ³J=6,9Hz, CH₃CHOH), 5,15 ppm (q, 2H, ³J=7,1Hz, CH).
**2.3 poly(benzyl glutamate)₈₀-propyl-poly(acide lactique)₃₀** : Du N-carboxyanhydride de L-glutamate de benzyle (9,74g, 37,0mmol) fourni par Flamel Technologies est pesé et introduit en boîte à gants sous atmosphère d'azote dans un ballon préalablement flammé. Le polylactide déprotégé (1g, 0,46mmol) est solubilisé dans du dichlorométhane fraîchement distillé (45ml) puis introduit par canule. Le milieu réactionnel est placé sous agitation magnétique pendant trois heures à température ambiante. Le solvant est évaporé au rotavapor puis le polymère est séché sous vide primaire. Rendement : 85%. *Caractérisations :* RMN ¹H (TFA d) : δ = 1,55 ppm (m, 6H, CH₃), 1,95 ppm (m, 2H, CH₂CH₂C=O), 2,35 ppm (m, 2H, CH₂CH₂C=O), 4,60 ppm (m, 1H, O=CCHNH), 5,0ppm (m, 2H, CH), 5,25 ppm (m, 2H, CH₂Ph), 7,10 ppm (m, 5H, Ph). RMN ¹³C (DMSO d6) : δ = 17 ppm (CH₃), 27 ppm (CH₂CH₂COO), 30 ppm (CH₂CH₂COO), 52 ppm (O=CCHNH), 66 ppm (CH₂Ph), 128-136 ppm (Ph), 168-170 ppm (OC=O), 172 ppm (NC=O).
**2.4 poly(acide glutamique)₈₀-propyl-poly(acide lactique)₃₀ :** Le copolymère (5g, 20,3mmol d'ester de benzyle) est introduit sous courant d'azote dans un ballon préalablement flammé. Il est solubilisé dans l'acide trifluoroacétique (44ml, 0,57mol). La solution est alors placée sous agitation à 10°C. On introduit l'acide méthane sulfonique (44ml, 0,68mol) et l'anisole (11 ml, 0,10mol) sous courant d'azote. On laisse le milieu réactionnel trois heures sous agitation à 10°C, puis on le précipite dans un large excès d'éther éthylique froid. Le polymère précipité est récupéré par filtration, lavé avec de l'éther éthylique et séché sous vide primaire. Rendement : 99%. *Caractérisations* :RMN ¹H (TFA d) : δ = 1,55 ppm (m, 6H, CH₃), 1,95 ppm (m, 2H, CH₂CH₂C=O), 2,35 ppm (m, 2H, CH₂CH₂C=O), 4,60 ppm (m, 1H, O=CCHNH), 5,0ppm (m, 2H, CH). RMN ¹³C (DMSO d6) : δ = 17 ppm (CH3), 27 ppm (CH₂CH₂COOH), 30 ppm (CH₂CH₂COOH), 52 ppm (OCCHNH), 69 ppm (CH), 168-170 ppm (O=CO), 172 ppm (O=CNH).

### Exemple 3 : poly(acide lactique)₅₀-bloc-(acide glutamique)₅₀

**3.1 BOC-aminopropyl-poly(acide lactique)₅₀** : Le L-lactide (5g, 34,70mmol, Aldrich 16101-127) est pesé et introduit en boîte à gants sous atmosphère d'azote dans un ballon préalablement flammé. Le toluène, fraîchement distillé (27ml) est introduit par canule dans le ballon que l'on place sous agitation magnétique pendant une heure à 80°C. Le ter-butoxycarbonyl aminopropanol (0,24g, 1,38mmol, Fluka 381029/1) et le toluène fraîchement distillé (23ml) sont introduits dans un ballon préalablement flammé et placé sous agitation magnétique dans un bain à -10°C. La solution de diéthylzinc dans le toluène (0,63ml, 0,69mmol, 1,1M, Aldrich 72560-099) est introduite goutte-à-goutte dans cette solution. Le milieu réactionnel est alors laissé sous agitation magnétique à température ambiante. Au bout d'une heure on introduit la solution de L-lactide sous courant d'azote dans le milieu réactionnel que l'on place alors à 80°C sous agitation pendant une heure. La polymérisation est terminée par un ajout de 4ml d'acide acétique en solution dans le toluène (10%). Le milieu réactionnel est alors concentré à l'évaporateur rotatif et précipité dans un large excès de méthanol froid. Le polymère précipité est récupéré par filtration puis séché sous vide primaire. Rendement : 98%. *Caractérisations :* Tg : 30-37°C. RMN ¹H (CDCl₃) : δ = 1,4 ppm (s, 9H, CCH₃), 1,55 ppm (d, 6H, ³J=7,1Hz, CHCH₃), 1,85 ppm (q, 2H, ³J=6,3Hz, CH₂CH₂CH₂), 3,1 ppm (q, 2H, ³J=6,2Hz, CH₂NH), 4,15 ppm (t, 2H, ³J=6,0Hz, CH₂O), 4,35 ppm (q, 1H, ³J=6,9Hz, CH₃CHOH), 4,8 ppm (m, 1H, NH), 5,15 ppm (q, 2H, ³J=7,1Hz, CHCH₃). RMN ¹³C (CDCl₃) : δ = 17 ppm (2C, CHCH₃), 20,7 ppm (1C, CH₃CHOH), 28,7 ppm (3C, CH₃C), 29,5 ppm (CH₂CH₂CH₂), 37,5 ppm (CH₂NH), 63,5 ppm (CH₂O), 67 ppm (CH₃CHOH), 69,5 ppm (2C, CH), 156 ppm (NHC=O), 170 ppm (CHC(O)O).
**3.2 aminopropyl-poly(acide lactigue)₅₀** : Le polylactide (4g, 1,11mmol) et le dichlorométhane fraîchement distillé (45ml) sont introduits sous courant d'azote dans un ballon préalablement flammé et relié à un bulleur. On introduit l'acide trifluoroacétique (8ml, 0,1mol, Sigma 19H3648) et on place la solution sous agitation à température ambiante pendant une demi-heure, jusqu'à ce que le dégagement de CO₂ soit terminé. On évapore les solvants du milieu réactionnel à l'évaporateur rotatif. On solubilise le polylactide dans 40ml de dichlorométhane. Cette phase organique est lavée deux fois par 40ml de NaHCO₃ en solution aqueuse (5%) puis deux fois par 40ml d'eau distillée jusqu'à ce que le pH des eaux de lavage soit neutre. La phase organique est alors séchée sur MgSO₄ puis filtrée. Le solvant est évaporé à l'évaporateur rotatif, puis le polymère est séché sous vide primaire. Rendement : 95%. *Caractérisations :* RMN ¹H (CDCl₃) : δ = 1,55 ppm (d, 6H, ³J=7,1Hz, CH3), 1,85 ppm (q, 2H, ³J=6,3Hz, CH₂CH₂CH₂), 2,8 ppm (q, 2H, ³J=6,2Hz, CH₂NH₂), 4,15 ppm (t, 2H, ³J=6,0Hz, CH₂O), 4,35 ppm (q, 1H, ³J=6,9Hz, CH₃CHOH), 5,15 ppm (q, 2H, ³J=7,1Hz, CH).
**3.3 poly(benzyl glutamate)₅₀-propyl-poly(acide lactique)₅₀** : Du N-carboxyanhydride de L-glutamate de benzyle (3,65g, 13,8mmol) est pesé et introduit en boîte à gants sous atmosphère d'azote dans un ballon préalablement flammé. Le polylactide déprotégé (1g, 0,27mmol) est solubilisé dans du dichlorométhane fraîchement distillé (17ml) puis introduit par canule. Le milieu réactionnel est placé sous agitation magnétique pendant trois heures à température ambiante. Le solvant est évaporé au rotavapor puis le polymère est séché sous vide primaire. Rendement : 85%. *Caractérisations :* RMN ¹H (TFA d) : δ = 1,55 ppm (m, 6H, CH₃), 1,95 ppm (m, 2H, CH₂CH₂C=O), 2,35 ppm (m, 2H, CH₂CH₂C=O), 4,60 ppm (m, 1H, O=CCHNH), 5,0ppm (m, 2H, CH), 5,25 ppm (m, 2H, CH₂Ph), 7,10 ppm (m, 5H, Ph).RMN ¹³C (DMSO d6) : δ = 17 ppm (CH₃), 27 ppm (CH₂CH₂COO), 30 ppm (CH₂CH₂COO), 52 ppm (O=CCHNH), 66 ppm (CH₂Ph), 128-136 ppm (Ph), 168-170 ppm (OC=O), 172 ppm (NC=O).
**3.4 poly(acide glutamique)₅₀-propyl-poly(acide lactique)₅₀:** Le copolymère (3g, 10,27mmol d'ester de benzyle) est introduit sous courant d'azote dans un ballon préalablement flammé. Il est solubilisé dans l'acide trifluorcacétique ((22,5ml, 0,29mol). La solution est alors placée sous agitation à 10°C. On introduit l'acide méthane sulfonique (22,5ml, 0,35mol) et l'anisole (5,5ml, 0,05mol) sous courant d'azote. On laisse le milieu réactionnel trois heures sous agitation à 10°C, puis on le précipite dans un large excès d'éther éthylique froid. Le polymère précipité est récupéré par filtration, lavé avec de l'éther éthylique et séché sous vide primaire. Rendement : 99%. *Caractérisations* : RMN ¹H (TFA d) : δ = 1,55 ppm (m, 6H, CH₃), 1,95 ppm (m, 2H, CH₂CH₂C=O), 2,35 ppm (m, 2H, CH₂CH₂C=O), 4,60 ppm (m, 1H, O=CCHNH), 5,0ppm (m, 2H, CH). RMN ¹³C (DMSO d6) : δ = 17 ppm (CH3), 27 ppm (CH₂CH₂COOH), 30 ppm (CH₂CH₂COOH), 52 ppm (OCCHNH), 69 ppm (CH), 168-170 ppm (O=CO), 172 ppm (O=CNH).

### Exemple 4 : poly(acide lactique)₈₀-bloc-(acide glutamique)₂₀

**4.1 BOC-aminopropyl-poly(acide lactique)₈₀:** L-lactide (5,2g, 36,09mmol, Aldrich 16101-127) et toluène distillé (27ml) sont introduits dans un ballon sec et sous azote. On chauffe une heure à 80°C. Dans un second ballon on prépare un mélange de t-butoxycarbonyl aminopropanol (0,16g, 0,91 mmol, Fluka 381029/1) et de toluène fraîchement distillé (23ml), qui est refroidi à -10°C. Après l'ajout du diéthylzinc (0,4ml, 0,44mmol, 1,1M dans le toluène, Aldrich 72560-099) au BOC-aminopropano, on permet à ce milieu réactionnel de devenir à température ambiante, puis on l'ajoute sur le monomère lactide pour initier la polymérisation. La polymérisation est terminée par un ajout de 0,5ml d'acide acétique en solution dans le toluène (10%). Le milieu réactionnel est alors concentré à l'évaporateur rotatif et précipité dans un large excès de méthanol. Le polymère est récupéré par filtration puis séché sous vide. Rendement : 98%. *Caractérisations :* Tg : 30-37°C. RMN ¹H (CDCl₃) : δ = 1,4 ppm (s, 9H, CCH₃), 1,55 ppm (d, 6H, ³J=7,1Hz, CHCH₃), 1,85 ppm (q, 2H, ³J=6,3Hz, CH₂CH₂,CH₂, 3,1 ppm (q, 2H, ³J=6,2Hz, CH₂NH), 4,15 ppm (t, 2H, ³J=6,0Hz, CH₂O), 4,35 ppm (q, 1H, ³J=6,9Hz, CH₃CHOH), 4,8 ppm (m, 1H, NH), 5,15 ppm (q, 2H, ³J=7,1Hz, CHCH₃). RMN ¹³C (CDCl₃) : δ = 17 ppm (2C, CHCH₃), 20,7 ppm (1C, CH₃CHOH), 28,7 ppm (3C, CH₃C), 29,5 ppm (CH₂CH₂CH2), 37,5 ppm (CH₂NH), 63,5 ppm (CH₂O), 67 ppm (CH₃CHOH), 69,5 ppm (2C, CH), 156 ppm (NHC=O), 170 ppm (CHC(O)O).
**4.2 aminopropyl-poly(acide lactique)₈₀:** Du polylactide (4,5g, 2,98mmol) et le dichlorométhane distillé (54ml) sont introduits sous courant d'azote dans un ballon. On introduit l'acide trifluoroacétique (9ml, 0,11 mol, Sigma 19H3648) et on place la solution sous agitation à température ambiante pendant une demi-heure, jusqu'à ce que le dégagement de CO₂ soit terminé. On évapore les solvants du milieu réactionnel à l'évaporateur rotatif. On ajoute 50ml de dichlorométhane et la solution est lavée deux fois par 50ml de NaHCO₃ en solution aqueuse (5%) puis deux fois par 50ml d'eau distillée jusqu'à ce que le pH des eaux de lavage soit neutre. La phase organique est séchée sur MgSO₄ puis filtrée. Le solvant est évaporé à l'évaporateur rotatif, puis le polymère est séché sous vide. Rendement : 95%. *Caractérisations :* RMN ¹H (CDCl₃) : δ = 1,55 ppm (d, 6H, ³J=7,1Hz, CH3), 1,85 ppm (q, 2H, ³J=6,3Hz, CH₂CH₂CH₂), 2,8 ppm (q, 2H, ³J=6,2Hz, CH₂NH₂), 4,15 ppm (t, 2H, ³J=6,0Hz, CH₂O), 4,35 ppm (q, 1H, ³J=6,9Hz, CH₃CHOH), 5,15 ppm (q, 2H, ³J=7,1 Hz, CH).
**4.3 poly(benzyl glutamate)₂₀-propyl-poly(acide lactique)₈₀:** Le N-carboxyanhydride de L-glutamate de benzyle (2,70g, 10,26mmol) est introduit dans un ballon. Le polylactide déprotégé (3g, 1,98mmol) est solubilisé dans du dichlorométhane fraîchement distillé (15ml) puis introduit par canule. Le milieu réactionnel est placé sous agitation magnétique pendant trois heures à température ambiante. Le solvant est évaporé au rotavapor puis le polymère est séché sous vide primaire. Rendement : 85%. *Caractérisations :* RMN ¹H (TFA d) : δ = 1,55 ppm (m, 6H, CH₃), 1,95 ppm (m, 2H, CH₂CH₂C=O), 2,35 ppm (m, 2H, CH₂CH₂C=O), 4,60 ppm (m, 1H, O=CCHNH), 5,0ppm (m, 2H, CH), 5,25 ppm (m, 2H, CH₂Ph), 7,10 ppm (m, 5H, Ph). RMN ¹³C (DMSO d6) : δ = 17 ppm (CH₃), 27 ppm (CH₂CH₂COO), 30 ppm (CH₂CH₂,COO), 52 ppm (O=CCHNH), 66 ppm (CH₂Ph), 128-136 ppm (Ph), 168-170 ppm (OC=O), 172 ppm (NC=O).
**4.4 poly(acide glutamique)₂₀-propyl-poly(acide lactique)₈₀** : Le copolymère (5,2g, 20,8mmol d'ester de benzyle) est introduit dans un ballon et solubilisé dans l'acide trifluoroacétique (22ml, 0,29mol) à 10°C. On introduit l'acide méthane sulfonique (22ml, 0,34mol) et l'anisole (5,6ml, 0,05mol) sous courant d'azote et on laisse le milieu réactionnel trois heures sous agitation à 10°C. Le polymère est précipité dans un large excès d'éther éthylique froid, récupéré par filtration, lavé avec de l'éther éthylique et séché sous vide. Rendement : 99%. *Caractérisations :* RMN ¹H (TFA d) : δ = 1,55 ppm (m, 6H, CH₃), 1,95 ppm (m, 2H, CH₂CH₂C=O), 2,35 ppm (m, 2H, CH₂CH₂C=O), 4,60 ppm (m, 1H, O=CCHNH), 5,0ppm (m, 2H, CH). RMN ¹³C (DMSO d6) : δ = 17 ppm (CH3), 27 ppm (CH₂CH₂COOH), 30 ppm (CH₂CH₂COOH), 52 ppm (OCCHNH), 69 ppm (CH), 168-170 ppm (O=CO), 172 ppm (O=CNH).

### Exemple 5 : poly(acide lactique)₃₀-bloc-(acide glutamique)₁₀₀

5.1 BOC-aminopropyl-poly(acide lactique)₃₀ Le L-lactide (6g, 41,64mmol, Aldrich 16101-127) est introduit en boîte à gants sous atmosphère d'azote dans un ballon préalablement flammé. Le toluène, fraîchement distillé (27ml) est introduit par canule dans le ballon que l'on place sous agitation magnétique pendant une heure à 80°C. Le ter-butoxycarbonyl aminopropanol (0,73g, 4,20mmol, Fluka 381029/1) et le toluène fraîchement distillé (23ml) sont introduits dans un ballon préalablement flammé et placé sous agitation magnétique dans un bain à -10°C. La solution de diéthylzinc dans le toluène (1,9ml, 2,19mmol, 1,1M, Aldrich 72560-099) est introduite goutte-à-goutte dans cette solution. Le milieu réactionnel est alors laissé sous agitation magnétique à température ambiante. Au bout d'une heure on introduit la solution de L-lactide sous courant d'azote dans le milieu réactionnel que l'on place alors à 80°C sous agitation pendant une heure. La polymérisation est terminée par un ajout de 5ml d'acide acétique en solution dans le toluène (10%). Le milieu réactionnel est alors concentré à l'évaporateur rotatif et précipité dans un large excès de méthanol froid. Le polymère précipité est récupéré par filtration puis séché sous vide primaire. Rendement : 98%. *Caractérisations :* Tg : 30-37°C. RMN ¹H (CDCl₃) : δ = 1,4 ppm (s, 9H, CCH₃), 1,55 ppm (d, 6H, ³J=7,1Hz, CHCH₃), 1,85 ppm (q, 2H, ³J=6,3Hz, CH₂CH₂CH₂), 3,1 ppm (q, 2H, ³J=6,2Hz, CH₂,NH). 4,15 ppm (t, 2H, ³J=6,0Hz, CH₂O), 4,35 ppm (q, 1H, ³J=6,9Hz, CH₃CHOH), 4,8 ppm (m, 1H, NH), 5,15 ppm (q, 2H, ³J=7,1Hz, CHCH₃). RMN ¹³C (CDCl₁₃) : δ = 17 ppm (2C, CHCH₃), 20,7 ppm (1C, CH₃CHOH), 28,7 ppm (3C, CH₃C), 29,5 ppm (CH₂CH₂CH₂), 37,5 ppm (CH₂NH), 63,5 ppm (CH₂O), 67 ppm (CH₃CHOH), 69,5 ppm (2C, CH), 156 ppm (NHC=O), 170 ppm (CHC(O)O).
**5.2 aminopropyl-poly(acide lactique)₃₀:** Le polylactide (3g, 1,39mmol) et le dichlorométhane fraîchement distillé (36ml) sont introduits sous courant d'azote dans un ballon préalablement flammé et relié à un bulleur. On introduit l'acide trifluoroacétique (6ml, 0,08mol, Sigma 19H3648) et on place la solution sous agitation à température ambiante pendant une demi-heure, jusqu'à ce que le dégagement de CO₂ soit terminé. On évapore les solvants du milieu réactionnel à l'évaporateur rotatif. On solubilise le polylactide dans 40ml de dichlorométhane. Cette phase organique est lavée deux fois par 40ml de NaHCO₃ en solution aqueuse (5%) puis deux fois par 40ml d'eau distillée jusqu'à ce que le pH des eaux de lavage soit neutre. La phase organique est alors séchée sur MgSO₄ puis filtrée. Le solvant est évaporé à l'évaporateur rotatif, puis le polymère est séché sous vide primaire. Rendement : 97%. *Caractérisations :* RMN ¹H (CDCl₃) : δ = 1,55 ppm (d, 6H, ³J=7,1Hz, CH3), 1,85 ppm (q, 2H, ³J=6,3Hz, CH₂CH₂CH₂), 2,8 ppm (q, 2H, ³J=6,2Hz, CH₂NH₂), 4,15 ppm (t, 2H, ³J=6,0Hz, CH₂O), 4,35 ppm (q, 1H, ³J=6,9Hz, CH₃CHOH), 5,15 ppm (q, 2H, ³J=7,1 Hz, CH).
**5.3 poly(benzyl glutamate)₁₀₀-propyl-poly(acide lactique)₃₀ :** Le N-carboxyanhydride de L-glutamate de benzyle (33g, 125,4mol) fourni par Flamel Technologies est pesé et introduit en boîte à gants sous atmosphère d'azote dans un ballon préalablement flammé. Le polylactide déprotégé (2,9g, 1,33mmol) est solubilisé dans du dichlorométhane fraîchement distillé (165ml) puis introduit par canule. Le milieu réactionnel est placé sous agitation magnétique pendant trois heures à température ambiante. Le solvant est évaporé au rotavapor puis le polymère est séché sous vide primaire. Rendement : 93%. *Caractérisations:* RMN ¹H (TFA d) : δ = 1,55 ppm (m, 6H, CH₃), 1,95 ppm (m, 2H, CH₂CH₂C=O), 2,35 ppm (m, 2H, CH₂CH₂C=0), 4,60 ppm (m, 1H, O=CCHNH), 5,0ppm (m, 2H, CH), 5,25 ppm (m, 2H, CH₂Ph), 7,10 ppm (m, 5H, Ph). RMN ¹³C (DMSO d6) : δ = 17 ppm (CH₃), 27 ppm (CH₂CH₂COO), 30 ppm (CH₂CH₂COO), 52 ppm (O=CCHNH), 66 ppm (CH₂Ph), 128-136 ppm (Ph), 168-170 ppm (OC=O), 172 ppm (NC=O).
**5.4 poly(acide glutamique)₁₀₀-propyl-poly(acide lactique)₃₀:** Le copolymère (11g, 44,66mmol d'ester de benzyle) est introduit sous courant d'azote dans un ballon préalablement flammé. II est solubilisé dans l'acide trifluoroacétique (100mil, 1,30mol). La solution est alors placée sous agitation à 10°C. On introduit l'acide méthane sulfonique (100ml, 1,55mol) et l'anisole (25ml, 0,23mol) sous courant d'azote. On laisse le milieu réactionnel trois heures sous agitation à 10°C, puis on le précipite dans un large excès d'éther éthylique froid. Le polymère précipité est récupéré par filtration, lavé avec de l'éther éthylique et séché sous vidé primaire. Rendement : 99%. *Caractérisations :* RMN ¹H (TFA d) : δ = 1,55 ppm (m, 6H, CH₃), 1,95 ppm (m, 2H, CH₂CH₂C=O), 2,35 ppm (m, 2H, CH₂CH₂C=O), 4,60 ppm (m, 1H, O=CCHNH), 5,0ppm (m, 2H, CH). RMN ¹³C (DMSO d6) _{:} δ = 17 ppm (CH3), 27 ppm (CH₂CH₂COOH), 30 ppm (CH₂CH₂COOH), 52 ppm (OCCHNH), 69 ppm (CH), 168-170 ppm (O=CO), 172 ppm (O=CNH).

### Exemple 6 : Formation de nanoparticules à partir du polymère de l'exemple 4

0.5 g de poudre du polymère de l'exemple 4 sont dissous dans 20 g d'un mélange 90/10 p/p THF/Ethanol. La solution est coulée au goutte à goutte dans 4 volumes d'une solution aqueuse de tampon phosphate 0.1 M. La dispersion obtenue est diffusante

### Exemple 7 : Mesure du diamètre hydrodynamique des nanoparticules de l'exemple 6

La dispersion diffusante de l'exemple 6 est diafiltrée sur une membrane BIOMAX YM 300. Les nanoparticules se trouvent concentrées dans le rétentat. Elles sont ensuite dialysées à volume constant contre 800 ml d'eau faiblement tamponnée (tampon phosphate 2.10⁻⁴M sans sel). Le diamètre hydrodynamique des particules, déterminé par diffusion de lumière dynamique, est de 240 nm.

### Exemple 8 : Mesure de la quantité maximale d'insuline absorbées sur les particules de polymère de l'exemple 7

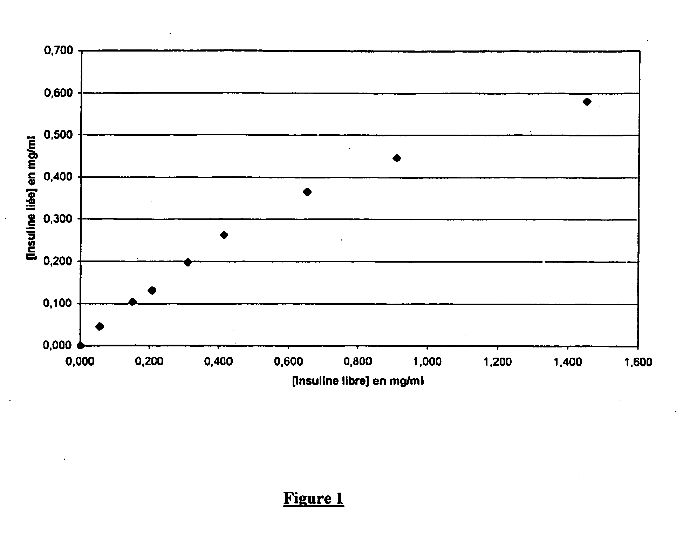

Les nanoparticules de l'exemple 7, concentrées à 10 mg/ml en conditions isotoniques à pH 7,4. sont mises en contact à 25°C durant 16 heures, avec des concentrations croissantes d'insuline recombinante humaine en solution. La quantité d'insuline libre c'est à dire non adsorbée sur les nanoparticules, est déterminée par chromatographie d'exclusion stérique. A cette fin, la préparation est injectée dans une colonne TSK G4000 PWXL de TOSO HAAS. L'insuline libre est détectée par un détecteur UV AGILENT Séries 1100 à 214 nm. On obtient ainsi l'isotherme d'adsorption de la figure 1. La valeur au plateau de cet isotherme permet de déterminer la quantité maximale, Am, d'insuline adsorbée par unité de masse du copolymère sec. On trouve Am = 6% p/p.

### Exemple 9 : Formation de nanoparticules à partir du polymère de l'exemple 5

Le polymère de l'exemple 5 est obtenu en solution éthanolique concentrée à 26.7 g/l. Cette solution est directement coulée au goutte à goutte dans 4 volumes d'une solution aqueuse de tampon phosphate 0.1 M. La dispersion obtenue est diffusante.

### Exemple 10 : Mesure du diamètre hydrodynamique des nanoparticules de l'exemple 9

La dispersion diffusante de l'exemple 9 est diafiltrée sur une membrane BIOMAX YM 300. Les nanoparticules se trouvent concentrées dans le retentat. Elles sont ensuite dialysées à volume constant contre 800 ml d'eau faiblement tamponnée (tampon phosphate 2.10-4 M sans sel). Le diamètre hydrodynamique des particules, déterminé par diffusion de lumière dynamique est de 220 nm.

### Exemple 11 : Mesure de la Quantité maximale d'insuline absorbées sur les particules de l'exemple 10

En procédant de manière identique à l'exemple 8, on obtient l'isotherme d'adsorption de l'insuline sur les nanoparticules du polymère de l'exemple 5. La valeur au plateau de cet isotherme permet de déterminer la quantité maximale, Am, d'insuline adsorbée par unité de masse du copolymère sec. On trouve Am = 1 % p/p.

### Exemple 12 : Caractérisation des nanoparticules du polymère de l'exemple 3

Les nanoparticules du copolymère de l'exemple 3 sont préparées et isolées selon le procédé exposé dans les exemples 6 et 9. Le diamètre hydrodynamique des nanoparticules, mesuré par diffusion de lumière dynamique, est de 450 nm. La quantité maximale d'insuline adsorbée sur ces nanoparticules est mesurée comme exposé dans les exemples 8 et 11. On trouve : Am = 2,5% p/p.

### Exemple 13 : Formation de nanoparticles à partir du polymère de l'exemple 3

500 mg polymère, selon l'exemple 3, sont dissous dans 100ml de DMF en 10 min à 60°C. Cette solution est versée lentement dans un volume de 200ml d'éther diisopropylique à -40°C fortement agité. La solution est laissée à reposer à température ambiante pendant 2 heures puis centrifugée à 1500 tr/min pendant 20 min. Le culot est filtre sur Büchner n°4 et le précipité est lavé avec de l'éther diisopropylique. Le précipité est séché sous vide de pompe à palettes.

### Exempte 14 : Pharmacocinétiaue et pharmacodynamie des PV-chargées avec l'insuline chez le chien sain à jeun.

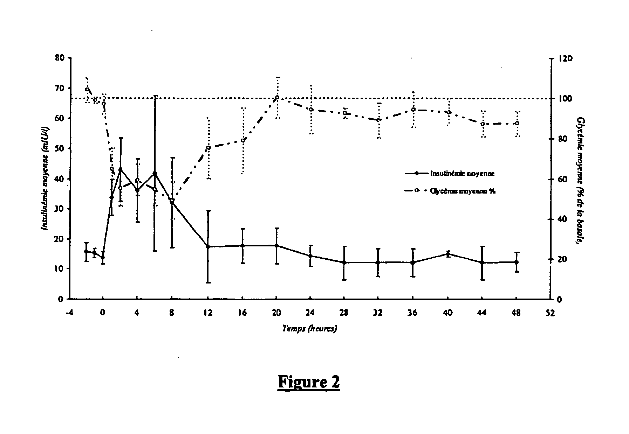

La préparation de microparticules PAHC-poly-AAI1 de l'exemple 7, associées à de l'insuline (Basulin®) de l'exemple 8 a été injectée à des chiens, rendus diabétiques par pancréatectomie totale, et à jeun de la veille au soir. L'administration à 11 heures du matin par voie sous cutanée thoracique de la préparation a été faite à la posologie de 0,5 UI/kg d'insuline par Kg de poids vif de l'animal. Le volume administré est compris entre 0,18 et 0,24 ml. Au temps -4, -2, 0, 1, 2, 4, 6, 8, 12, 16, 20, 24, 28, 32, 36, 40, 44 et 48 heures, 1 ml de sang est prélevé par ponction, jugulaire sous vide sur tube héparinate de sodium. 30 µl de sang total sont utilisés extemporanément pour mesure de la glycémie. Le tube est ensuite centrifugé, décanté et le plasma stocké à -20° C pour dosage de l'insuline. Les résultats présentés dans la figure 2 ci-après montrent un relarguage de l'insuline jusqu'à 12 heures (trait plein) et un effet hypoglycémiant important qui se prolonge jusqu'à 20 heures (trait discontinu) après l'injection.

Cet exemple démontre la non-dénaturation de l'insuline en présence de PV selon l'invention.

De plus, cet exemple montre que les nanoparticules selon l'invention sont de PV peuvent être utilisées efficacement pour la libération modifiée de protéines.

## Revendications

1. Suspension colloïdale de particules submicroniques susceptibles d'être utilisées, notamment pour la vectorisation de principe(s) actif(s) (PA), ces particules étant des arrangements supramoléculaires individualisés, à base d'un copolymère amphiphile comprenant :
► au moins un bloc de polyaminoacide(s) (PAA) linéaire(s), hydrophile(s) à enchaînements α-peptidiques, les aminoacides hydrophiles AAI constitutifs de ce bloc PAA étant identiques ou différents entre eux ;
► et au moins un bloc d'au moins un polymère hydrophobe, formé par un Polymère d'Acide α-HydroxyCarboxylique (PAHC) - de préférence Polymère d'Acide Lactique (PAL)ou Polymère d'Acide Glycolique (PAG)-
**caractérisée en ce que** :
- elle peut être obtenue spontanément en l'absence de tensioactif, par mise en présence de copolymère amphiphile avec un liquide non-solvant du copolymère amphiphile;
- elle est stable même en l'absence de tensioactif ;
- les AAI du copolymère sont au moins partiellement sous forme ionisée ;
- les particules sont aptes à s'associer en suspension colloïdale à l'état non dissous, avec au moins un PA et à libérer celui-ci, notamment in vivo, de manière prolongée et/ou retardée.

2. Suspension selon la revendication 1, **caractérisée en ce qu'**elle est obtenue par mise en solution du copolymère amphiphile dans un solvant organique et mise en présence de cette solution avec un liquide aqueux.

3. Suspension selon la revendication 1 ou 2, **caractérisée en ce que** :
- les AAI sont des acides aminés hydrophiles AAI,
- le rapport AHC/AAI est supérieur à 0,1
- la longueur absolue du bloc PAHC est supérieure à 2 monomères, de préférence supérieure à 10 monomères, et plus préférentiellement comprise entre 20 et 60 monomères.

4. Suspension selon l'une quelconque des revendications 1 a 3, **caractérisée en ce que** le ou les blocs PAA à base d'AAI en comprennent au moins 5, de préférence au moins 20, et plus préférentiellement encore entre 30 et 100.

5. Suspension selon l'une quelconque des revendications 1 à 4, **caractérisée en ce que** les particules sont des « diblocs » PAHC/AAI.

6. Suspension selon l'une quelconque des revendications 1 à 5, **caractérisée en ce que** le(ou les)AAI est(sont) choisi(s) parmi des aminoacides à chaîne latérale ionisable, les aminoacides naturels Glu et Asp sous forme carboxylique et/ou sous forme de sels étant particulièrement préférés.

7. Suspension selon l'une quelconque des revendications 1 à 6, **caractérisée en ce qu'**elle est aqueuse et stable.

8. Solide pulvérulent, **caractérisé en ce qu'**il est obtenu à partir de la suspension selon l'une quelconque des revendications 1 à 7.

9. Procédé de préparation du solide pulvérulent selon la revendication 8, **caractérisée en ce que** :
- 1)- on met en oeuvre ou on prépare par polymérisation de monomères d'acide(s) α-hydroxycarboxylique(s)-de préférence acide lactique ou glycolique- au moins un bloc PAHC (de préférence de PAL ou PAG); ce bloc PAHC étant fonctionnalisé (avantageusement à au moins l'une de ses extrémités) par au moins un groupement réactif protecteur, de préférence choisi dans le groupe comprenant la BOC-éthanolamine, BOC-aminopropanol (BOC = ButOxyCarbonyle) ;
- 2)- on déprotège le bloc PAHC de l'étape -1)- ;
- 3)- on réalise une copolymérisation de monomères formés par des anhydrides de N-CarboxyAminoacides (NCA) d'amino-acides hydrophiles AAI et/ou par des anhydrides de N-CarboxyAminoacides (NCA) précurseurs d'amino-acides hydrophiles AAI et porteurs de groupements protecteurs, en présence d'au moins un solvant organique, de préférence choisi dans le groupe comprenant : la N-MéthylPyrrolidone (NMP), le DiMéthylFormamide (DMF), le DiMéthylsulfOxyde (DMSO), le DiMéthylAcétamide (DMAc), la pyrrolidone et le dichlorométhane ; ce dernier étant plus particulièrement préféré ;
- 4)- on ajoute le bloc PAHC déprotégé de l'étape -2)- au milieu de polymérisation du bloc de poly-AAI, avant, pendant ou après la polymérisation;
- 5)- éventuellement on déprotège les précurseurs d'amino-acides hydrophiles AAI pour obtenir un ou plusieurs blocs polyAAI ;
- 6)- on précipite le copolymère bloc PAHC-polyAAI obtenu à l'issue des étapes précédentes ;
- 7)- on met en solution le précipité de copolymère bloc PAHC-polyAAI obtenu à l'étape -6)- et on met en présence cette solution avec un liquide contenant au moins un non-solvant du copolymère bloc PAHC-polyAAI, de préférence l'eau, ce liquide ayant un pH choisi de telle sorte que les AAI du copolymère bloc PAHC-polyAAI soient au moins en partie ionisés ;
- 8)- éventuellement on associe au moins un principe actif PA hydrophile avec les particules ;
- 9)- éventuellement on purifie la suspension de l'étape -7)- ;
- 10)- éventuellement on concentre la suspension de l'étape -7)- ;
- 11)- on élimine le milieu liquide pour recueillir le solide pulvérulent comprenant les particules.

10. Procédé selon la revendication 9, **caractérisé en ce que**, le (ou les )bloc(s) PAHC fonctionnalisé(s) est (sont) introduit(s) avant et/ou au début de la polymérisation, qui se déroule de préférence à une température comprise entre 20 et 120°C à pression atmosphérique normale.

11. Procédé de préparation d'une suspension **caractérisé en ce que** l'on met en présence d'un milieu aqueux non-solvant du copodymère amphipile, solide pulvérulent selon la revendication 8 et/ou le solide pulvérulent obtenu par le procédé selon la revendication 9 ou 10.

12. Procédé de préparation d'une suspension , **caractérisé en ce qu'**il comprend les étapes 1 à 7 et éventuellement 8 et/ou 9 et/ou 10 du procédé selon la revendication 9.

13. Procédé de préparation d'une suspension , **caractérisé en ce que** l'on effectue l'association du PA hydrophile aux particules, par mise en présence d'une phase liquide contenant ledit PA hydrophile avec la suspension colloïdale de particules, telle que définie dans l'une quelconque des revendications 1 à 7.

14. Procédé de préparation d'une suspension , **caractérisé en ce que** l'on effectue l'association du PA hydrophile aux particules par mise en présence dudit PA à l'état solide avec la suspension colloïdale de particules, tellle que définie dans l'une quelconque des revendications 1 à 7.

15. Procédé de préparation d'une suspension **caractérisé en ce que** l'on met en présence le solide pulvérulent selon la revendication 8 et/ou le solide pulvérulent obtenu par le procédé selon la revendication 9, avec une phase liquide contenant le PA hydrophile.

16. Procédé de préparation d'une suspension , **caractérisé en ce que** l'on met en présence le solide pulvérulent selon la revendication 8 et/ou le solide pulvérulent obtenu par le procédé selon la revendication 9, avec le PA hydrophile sous forme solide et **en ce que** l'on disperse ce mélange de solides dans une phase liquide, de préférence une solution aqueuse.

17. Produits intermédiaires du procédé selon la revendication 9 ou 10, **caractérisés en ce qu'**ils sont constituées par des copolymères PAHC-polyAAI, de préférence pdy(acide lactique)-polyAAI ou poly(acide glycolique) polyAAI.

18. Suspension selon l'une quelconque des revendications 1 à 7 et/ou solide pulvérulent selon la revendication 8 comprenant un moins un principe actif hydrophile choisi, de préférence, parmi :
o les vaccins ;
o les protéines et/ou les peptides, parmi lesquels les plus préférentiellement retenus sont : les hémoglobines, les cytochromes, les albumines, les interférons, les antigènes, les anticorps, l'érythropoïétine, l'insuline, les hormones de croissance, les facteurs VIII et IX, les interleukines ou leurs mélanges, les facteurs stimulants de t'hématopoïèse ;
o les polysaccharides, l'héparine étant plus particulièrement sélectionnée ;
o les acides nucléiques et, préférablement, les oligonucléotides d'ARN et/ou d'ADN ;
o des molécules non peptido-protéiques appartenant à diverses classes de chimiothérapie anti-cancéreuses et, en particulier, les anthracyclines et les taxoïdes ;
o et leurs mélanges.

19. Spécialité pharmaceutique, nutritionnelle, phytosanitaire ou cosmétique, **caractérisée en ce qu'**elle comporte une suspension selon l'une quelconque des revendications 1 à 7 et/ou du solide pulvérulent selon la revendication 8.

## Claims

1. A colloidal suspension of submicronic particles which can be used in particular for delivering active principle(s) (APs), these particles being individualized supramolecular arrangements based on an amphiphilic copolymer including:
► at least one block of α-peptide-linked hydrophilic linear polyamino acid(s) (PAAs), the hydrophilic amino acids AAI constituting this PAA block being identical to or different from one another;
► and at least one block of at least one hydrophobic polymer, made of a poly(α-hydroxycarboxylic acid) (PHCA) - preferably poly(lactic acid) (PLA) or poly(glycolic acid) (PGA) -
**characterized in that**:
- it can be obtained spontaneously in the absence of surfactant, by bringing together the amphiphilic copolymer and a liquid that is not a solvent for the amphiphilic copolymer;
- it is stable even in the absence of surfactants;
- the AAIs of the copolymer are at least partially in ionized form;
- the particles are capable of associating in suspension in the nondissolved state with at least one **AP** and of releasing it, in particular in vivo, in a sustained and/or delayed manner.

2. The suspension according to claim 1, **characterized in that** it is obtained by dissolving the amphiphilic copolymer in an organic solvent and bringing together this solution and an aqueous liquid.

3. The suspension according to claim 1 or 2, **characterized in that**
- the AAIs are hydrophilic amino acids AAI;
- the HCA/AAI ratio is greater than 0.1;
- the absolute length of the PHCA block is greater than 2 monomers, preferably greater than 10 monomers, and more preferably between 20 and 60 monomers.

4. The suspension according to any one of claims 1 to 3, **characterized in that** the PAA blocks based on AAI include at least 5, preferably at least 20, and even more preferably between 30 and 100 thereof.

5. The suspension according to any one of claims 1 to 4, **characterized in that** the particles are PHCA/AAI "diblocks".

6. The suspension according to any one of claims 1 to 5, **characterized in that** the AAI(s) is (are) chosen from amino acids with an ionizable side chain, the natural amino acids Glu and Asp in carboxylic form and/or in the form of salts being particularly preferred.

7. The suspension according to any one of claims 1 to 6, **characterized in that** it is aqueous and stable.

8. A pulverulent solid, **characterized in that** it is obtained from the suspension according to any one of claims 1 to 7.

9. A method for preparing the pulverulent solid according to claim 8, **characterized in that**:
- 1)- at least one PHCA (preferably PLA or PGA) block is used or prepared by polymerization of monomers of a-hydroxycarboxylic acid(s), preferably lactic acid or glycolic acid; this PHCA block being functionalized (advantageously at at least one of its ends) with at least one protective reactive group, preferably chosen from the groups comprising BOC-ethanolamine and BOC-aminopropanol (BOC = ButOxyCarbonyl);
- 2)- the PHCA block of step -1)- is deprotected;
- 3)- a copolymerization of monomers made of AAI hydrophilic amino acid N-carboxyamino acid anhydrides (NCAs) and/or of AAI hydrophilic amino acid-precursor N-carboxyamino acid anhydrides (NCAs), carrying protective groups, is carried out in the presence of at least one organic solvent, preferably chosen from the group comprising: N-methylpyrrolidone (NMP), dimethylformamide (DMF), dimethyl sulfoxide (DMSO), dimethylacetamide (DMAc), pyrrolidone and dichloromethane, the latter being more particularly preferred;
- 4)- the deprotected PHCA block of step -2)- is added to the polyAAI block polymerization medium, before, during or after the polymerization;
- 5)- optionally, the AAI hydrophilic amino acid precursors are deprotected so as to obtain one or more polyAAI blocks;
- 6)- the PHCA-polyAAI block copolymer obtained at the end of the preceding steps is precipitated;
- 7)- the PHCA-polyAAI block copolymer precipitate obtained in step -6)- is dissolved and this solution is brought together with a liquid containing at least one non-solvent for the PHCA-polyAAI block copolymer, preferably water, this liquid having a pH chosen such that the AAIs of the PHCA-polyAAI block copolymer are at least partly ionized;
- 8)- optionally, at least one hydrophilic active principle **AP** is associated with the particles;
- 9)- optionally, the suspension of step -7)- is purified;
- 10)- optionally, the suspension of step -7)- is concentrated;
- 11)- the liquid medium is eliminated so as to collect the pulverulent solid comprising the particles.

10. The method according to claim 9, **characterized in that** the functionalized PHCA block(s) is (are) introduced before and/or at the beginning of the polymerization, which preferably takes place at a temperature of between 20 and 120°C at normal atmospheric pressure.

11. A method for preparing a suspension, **characterized in that** an aqueous medium that is not a solvent for the amphiphilic copolymer is placed together with the pulverulent solid according to claim 8 and/or the pulverulent solid obtained by means of the method according to claim 9 or 10.

12. A method for preparing a suspension, **characterized in that** it includes steps 1 to 7 and, optionally, 8 and/or 9 and/or 10 of the method according to claim 9.

13. A method for preparing a suspension, **characterized in that** the association of the hydrophilic **AP** with the particles is carried out by bringing together a liquid phase containing said hydrophilic **AP** and the colloidal suspension of particles, as claimed in any one of claims 1 to 7.

14. A method for preparing a suspension, **characterized in that** the association of the hydrophilic **AP** with the particles is carried out by bringing together said **AP** in the solid state and the colloidal suspension of particles, as claimed in any one of claims 1 to 7.

15. A method for preparing a suspension, **characterized in that** the pulverulent solid according to claim 8 and/or the pulverulent solid obtained by means of the method according to claim 9, and the liquid phase containing the hydrophilic **AP**, are brought together.

16. A method for preparing a suspension, **characterized in that** the pulverulent solid according to claim 8 and/or the pulverulent solid obtained by means of the method according to claim 9, and the hydrophilic **AP** in solid form, are brought together, and **in that** this mixture of solids is dispersed in a liquid phase, preferably in aqueous solution.

17. An intermediate product of the method according to claim 9 or 10, **characterized in that** it consists of PHCA-polyAAI, preferably poly(lactic acid)-polyAAI or poly(glycolic acid)-polyAAI, copolymers.

18. The suspension according to any one of claims 1 to 7 and/or the pulverulent solid according to claim 8, including at least one hydrophilic active principle preferably chosen from:
o vaccines;
o proteins and/or peptides, among which those most preferably selected are: hemoglobins, cytochromes, albumins, interferons, cytokines, antigens, antibodies, erythropoietin, insulin, growth hormones, factors VIII and IX, interleukins or mixtures thereof, hematopoiesis-stimulating factors;
o polysaccharides, heparin being more particularly selected;
o nucleic acids, and preferably RNA or DNA oligonucleotides;
o non-peptido-protein molecules belonging to various anticancer chemotherapy classes, and in particular anthracyclins and taxoids;
o and mixtures thereof.

19. A pharmaceutical, nutritional, plant-care or cosmetic specialty product, **characterized in that** it includes a suspension according to any of one of claims 1 to 7 and/or pulverulent solid according to claim 8.

## Patentansprüche

1. Kolloidale Suspension von Submikron-Partikeln, die insbesondere für die Vektorisierung von (einem) Wirkstoff(en) (WS) verwendet werden können, wobei es sich bei diesen Partikeln um individualisierte supramolekulare Anordnungen auf Basis eines amphiphilen Copolymers handelt, umfassend:
► mindestens einen Block von gerade(r/n), hydrophile(r/n) Polyaminosäure(n) (PAA) mit α-Peptidverknüpfungen, wobei die hydrophilen Aminosäuren AAI, die diesen PAA-Block bilden, untereinander verschieden oder gleich sind;
► und mindestens einen Block aus mindestens einem hydrophoben Polymer, das durch ein Polymer von α-Hydroxycarbonsäure (PHCA) - vorzugsweise ein Polymer von Milchsäure (PLA) oder ein Polymer von Glykolsäure (PGA) - gebildet wird,
**dadurch gekennzeichnet, dass**:
- sie spontan in Abwesenheit eines oberflächenaktiven Mittels erhalten werden kann, indem man das amphiphile Copolymer mit einer Nicht-Lösungsmittel-Flüssigkeit des amphiphilen Copolymers vereinigt;
- sie auch in Abwesenheit eines oberflächenaktiven Mittels stabil ist;
- die AAI des Copolymers zumindest teilweise in ionisierter Form vorliegen;
- die Partikel derart ausgelegt sind, dass sie sich in kolloidaler Suspension im ungelösten Zustand mit mindestens einem WS zusammenlagern und diesen, insbesondere in vivo, auf verlängerte und/oder verzögerte Weise freisetzen.

2. Suspension nach Anspruch 1, **dadurch gekennzeichnet, dass** sie, durch die Einbringung des amphiphiles Copolymers in einem organischen Lösungsmittel und durch die Vereinigung dieser Lösung mit einer wässrigen Flüssigkeit, erhalten wird.

3. Suspension nach Anspruch 1 oder 2, **dadurch gekennzeichnet, dass**:
- die AAI hydrophile Aminosäuren AAI sind,
- das Verhältnis HCA/AAI größer als 0,1 ist,
- die absolute Länge des PHCA-Blocks größer als 2 Monomere, vorzugsweise größer als 10 Monomere ist und stärker bevorzugt zwischen 20 und 60 Monomere beträgt.

4. Suspension nach einem der Ansprüche 1 bis 3, **dadurch gekennzeichnet, dass** der PAA-Block oder die PAA-Blöcke auf Basis von AAI mindestens 5, vorzugsweise mindestens 20 und noch stärker bevorzugt zwischen 30 und 100 AAI umfassen.

5. Suspension nach einem der Ansprüche 1 bis 4, **dadurch gekennzeichnet, dass** es sich bei den Partikeln um PHCA/AAI-"Diblöcke" handelt.

6. Suspension nach einem der Ansprüche 1 bis 5, **dadurch gekennzeichnet, dass** die AAI aus Aminosäuren mit ionisierbarer Seitenkette ausgewählt ist/sind, wobei die natürlichen Aminosäuren Glu und Asp in der Carboxylform und/oder der Salzform besonders bevorzugt sind.

7. Suspension nach einem der Ansprüche 1 bis 6, **dadurch gekennzeichnet, dass** sie wässrig und stabil ist.

8. Pulverförmiger Feststoff, **dadurch gekennzeichnet, dass** er ausgehend von der Suspension nach einem der Ansprüche 1 bis 7 erhalten wird.

9. Verfahren zur Herstellung des pulverförmigen Feststoffs nach Anspruch 8, **dadurch gekennzeichnet, dass** man:
- 1)- mindestens ein PHCA- (vorzugsweise PLA- oder PGA-) Block verwendet oder durch Polymerisation von Monomeren von α-Hydroxycarbonsäure(n) - vorzugsweise Milch- oder Glykolsäure - herstellt; wobei dieser PHCA-Block (vorzugsweise an mindestens einem seiner Enden) mit mindestens einer reaktiven Schutzgruppe funktionalisiert ist, die vorzugsweise aus der Gruppe ausgewählt ist, die BOC-Ethanolamin, BOC-Aminopropanol (BOC = Butoxycarbonyl) umfasst;
- 2)- die Schutzgruppen von dem PHCA-Block aus Schritt -1)- entfernt;
- 3)-eine Copolymerisation von Monomeren durchführt, die durch Anhydride von N-Carboxyaminosäuren (NCA) von hydrophilen Aminosäuren AAI und/oder durch Anhydride von N-Carboxyaminosäuren (NCA), die Vorstufen von hydrophilen Aminosäuren AAI sind und Schutzgruppen tragen, gebildet werden, in Gegenwart von mindestens einem organischen Lösungsmittel durchführt, das vorzugsweise aus der Gruppe ausgewählt wird, die Folgende umfasst: N-Methylpyrrolidon (NMP), Dimethylformamid (DMF), Dimethylsulfoxid (DMSO), Dimethylacetamid (DMAc), Pyrrolidon und Dichlormethan, wobei diese Letzteren ganz besonders bevorzugt sind;
- 4)- das PHCA-Block ohne Schutzgruppe aus Schritt -2)- zu dem Polymerisationsmedium des Poly-AAI-Blocks vor, während oder nach der Polymerisation zugibt;
- 5)- gegebenenfalls die Schutzgruppen von den Vorstufen von hydrophilen Aminosäuren AAI entfernt, um einen oder mehrere PolyAAI-Blöcke zu erhalten;
- 6)- das nach den vorhergehenden Schritten erhaltene PHCA-PolyAAI-Block-Copolymer ausfällt;
- 7)- den im Schritt -6)- erhaltenen Niederschlag des PHCA-PolyAAI-Block-Copolymers in Lösung bringt und diese Lösung mit einer Flüssigkeit vereinigt, die mindestens ein Nicht-Lösungsmittel für das PHCA-PolyAAI-Block-Copolymer, vorzugsweise Wasser, enthält, wobei diese Flüssigkeit einen pH hat, der derart gewählt wird, dass die AAI des PHCA-PolyAAI-Block-Copolymers zumindest teilweise ionisiert sind;
- 8)- gegebenenfalls mindestens einen hydrophilen Wirkstoff WS mit den Partikeln zusammenlagert;
- 9)- gegebenenfalls die Suspension von Schritt -7)- reinigt;
- 10)- gegebenenfalls die Suspension von Schritt -7)- konzentriert;
- 11)- das wässrige Medium beseitigt, um den pulverförmigen Feststoff, der die Partikel umfasst, zu erhalten.

10. Verfahren nach Anspruch 9, **dadurch gekennzeichnet, dass** der (oder die) funktionalisierte(n) PHCA-Block (-Blöcke) vor und/oder zu Beginn der Polymerisation, die vorzugsweise bei einer Temperatur zwischen 20 und 120°C bei normalem Atmosphärendruck abläuft, eingebracht wird (werden).

11. Verfahren zur Herstellung einer Suspension, **dadurch gekennzeichnet, dass** man den pulverförmigen Feststoff nach Anspruch 8 und/oder den pulverförmigen Feststoff, der durch das verfahren nach Anspruch 9 oder 10 erhalten wird, mit einem wässrigen Medium vereinigt, das ein Nicht-Lösungsmittel des amphiphilen Copolymers ist.

12. Verfahren zur Herstellung einer Suspension, **dadurch gekennzeichnet, dass** es die Schritte 1 bis 7 und gegebenenfalls 8 und/oder 9 und/oder 10 des Verfahrens nach Anspruch 9 umfasst.

13. Verfahren zur Herstellung einer Suspension, **dadurch gekennzeichnet, dass** man die Anlagerung des hydrophilen WS an die Partikel durchführt, indem man eine flüssige Phase, die den hydrophilen WS enthält, mit der kolloidalen Suspension von Partikeln, wie in einem der Ansprüche 1 bis 7 definiert, vereinigt.

14. Verfahren zur Herstellung einer Suspension, **dadurch gekennzeichnet, dass** man die Anlagerung des hydrophilen WS an die Partikel durchführt, indem man den WS in festem Zustand mit der kolloidalen Suspension von Partikeln, wie in einem der Ansprüche 1 bis 7 definiert, vereinigt.

15. Verfahren zur Herstellung einer Suspension, **dadurch gekennzeichnet, dass** man den pulverförmigen Feststoff nach Anspruch 8 und/oder den pulverförmigen Feststoff, der durch das Verfahren nach Anspruch 9 erhalten wird, mit einer flüssigen Phase, die den hydrophilen WS enthält, vereinigt.

16. Verfahren zur Herstellung einer Suspension, **dadurch gekennzeichnet, dass** man den pulverförmigen Feststoff nach Anspruch 8 und/oder den pulverförmigen Feststoff, der durch das Verfahren nach Anspruch 9 erhalten wird, mit dem hydrophilen WS in fester Form vereinigt, und **dadurch**, dass man dieses Gemisch von Feststoffen in einer flüssigen Phase, vorzugsweise einer wässrigen Lösung, dispergiert.

17. Zwischenprodukte des Verfahrens nach Anspruch 9 oder 10, **dadurch gekennzeichnet, dass** sie aus den PHCA-PolyAAI-Copolymeren, vorzugsweise Poly(Milchsäure)-PolyAAI oder Poly(Glykolsäure)-PolyAAI, bestehen.

18. Suspension nach einem der Ansprüche 1 bis 7 und/oder pulverförmiger Feststoff nach Anspruch 8, umfassend mindestens einen hydrophilen Wirkstoff, der vorzugsweise ausgewählt ist aus:
o Impfstoffen,
o Proteinen und/oder Peptiden, unter denen die Folgenden ganz besonders bevorzugt verwendet werden: Hämoglobine, Cytochrome, Albumine, Interferone, Antigene, Antikörper, Erythropoetin, Insulin, Wachstumshormone, die Faktoren VIII und IX, Interleukine oder ihre Gemische, Hämatopoesestimulierende Faktoren;
o Polysaccharide, wobei Heparin ganz besonders bevorzugt ausgewählt wird;
o Nukleinsäuren und vorzugsweise RNA- und/oder DNA-Oligonukleotide;
o Nicht-Peptid-Proteinmoleküle, die zu verschiedenen Antikrebs-Chemotherapie-Klassen gehören, und insbesondere Anthracycline und Taxoide
o und ihre Gemische.

19. Pharmazeutische, nahrungsergänzende, phytosanitäre oder kosmetische Spezialität, **dadurch gekennzeichnet, dass** sie eine Suspension nach einem der Ansprüche 1 bis 7 und/oder pulverförmigen Feststoff nach Anspruch 8 beinhaltet.
